# EUROPEAN PATENT SPECIFICATION

(11) **EP 1 708 746 B1**
(45) Date of publication and mention of the grant of the patent: **04.08.2010**
(21) Application number: 04816681.3
(22) Date of filing: 30.12.2004
(51) Int. Cl.: A61K 39/12, C12N 15/861, C07K 14/18

(54) **RECOMBINANT VACCINE AGAINST JAPANESE ENCEPHALITIS VIRUS [JEV] INFECTION AND A METHOD THEREOF**
REKOMBINANTE VAKZINE GEGEN INFEKTION MIT DEM JAPANISCHEN ENZEPHALITIS-VIRUS [JEV] UND VERFAHREN DAVON
VACCIN RECOMBINANT DIRIGE CONTRE L'INFECTION PAR LE VIRUS DE L'ENCEPHALITE JAPONAISE [JEV] ET PROCEDE CORRESPONDANT

(30) Priority: 04.01.2004 IN DE15162003
(43) Date of publication of application: 11.10.2006
(73) Proprietor: National Institute of Immunology, New Delhi 110067 (IN)
(72) Inventor: VRATI, Sudhanshu, National Institute of Immunology, New Delhi 110 067 (IN)
(74) Representative: Ebner von Eschenbach, Jennifer
(86) International application number: PCT/IN2004/000432
(87) International publication number: WO 2005/065707

(56) References cited:
- WO-A-96/37221
- US-A- 5 494 671
- JAISWAL SMITA ET AL: "Replication-defective adenoviral vaccine vector for the induction of immune responses to dengue virus type 2." JOURNAL OF VIROLOGY, vol. 77, no. 23, December 2003 (2003-12), pages 12907-12913, XP002331687 ISSN: 0022-538X
- JAN LEI-RON ET AL: "Increased immunogenicity and protective efficacy in outbred and inbred mice by strategic carboxyl-terminal truncation of Japanese encephalitis virus envelope glycoprotein" AMERICAN JOURNAL OF TROPICAL MEDICINE AND HYGIENE, vol. 48, no. 3, 1993, pages 412-423, XP008048530 ISSN: 0002-9637 cited in the application
- KAUR RUPINDERJEET ET AL: "Plasmid DNA immunization against Japanese encephalitis virus: immunogenicity of membrane-anchored and secretory envelope protein." THE JOURNAL OF INFECTIOUS DISEASES. 1 JAN 2002, vol. 185, no. 1, 1 January 2002 (2002-01-01), pages 1-12, XP002331689 ISSN: 0022-1899 cited in the application
- SWAMINATHAN S ET AL: "Viral vaccines for dengue: The present and the future" DENGUE BULLETIN 2003 INDIA, vol. 27, 2003, pages 181-191, XP002331688 ISSN: 1020-895X
- STEPHENSON J: "Defective adenoviruses as novel vaccines for the Flaviviridae." CLINICAL AND DIAGNOSTIC VIROLOGY. 15 JUL 1998, vol. 10, no. 2-3, 15 July 1998 (1998-07-15), pages 187-194, XP002331686 ISSN: 0928-0197
- KINNEY RICHARD M ET AL: "Development of new vaccines against dengue fever and Japanese encephalitis" INTERVIROLOGY, vol. 44, no. 2-3, March 2001 (2001-03), pages 176-197, XP008041380 ISSN: 0300-5526
- APPIAIHGARI M ET AL.: "Immunization with recombinant adenovirus synthesizing secretory form of Japanese Encephalitis Virus envelope protein protects mice against lethal encephalitis" INTERNET, [Online] October 2004 (2004-10), page 53, XP002331690 Retrieved from the Internet: URL:http://www.pdvi.org/PDFs/ABSTRACT_BOOK _FIRST_ASIAN.pdf> [retrieved on 2005-06-13]

## Description

### Field of the invention

The present invention relates to a method of preparing a novel recombinant adenovirus (RAdEs) vaccine to protect against Japanese encephalitis virus (JEV) infection. Also, it relates to a method of immunization and to the vaccine *per se.*

### Background and prior art references of the invention

Japanese encephalitis virus (JEV) is a member of the *flaviviridae* family of animal viruses that consists of several viruses of immense medical significance such as those causing dengue and yellow fever. JEV, transmitted to human beings by mosquitoes, is responsible for an acute infection of the central nervous system resulting in encephalitis. The virus is active over a vast geographic area covering India, China, Japan and virtually all of the South-East Asia. Approximately 3 billion people live in JEV endemic area and up to 50,000 cases of JEV infection are reported every year, of which, about 10,000 cases result in fatality and a high proportion of survivors end up having serious neurological and psychiatric sequel (45). A mouse brain-grown, formalin-inactivated JEV vaccine is available internationally. However, this vaccine has limitations in terms of its high cost of production, lack of long-term immunity and risk of allergic reaction due to the presence of the murine encephalogenic basic proteins or gelatin stabilizer (1, 33, 38, 39). There is, thus, an urgent need to develop an improved vaccine against JEV and several potential vaccines are currently being investigated in various laboratories (16).

JEV contains a single-stranded, plus-sense RNA genome of ∼11 Kb. It consists of a single open reading frame that codes for a large polyprotein of 3432 amino acids which is co- and post-translationally cleaved into three structural (capsid, C; pre-membrane, prM; and envelope, E) and seven non-structural proteins (NS1, NS2A, NS2B, NS3, NS4A, NS4B and NS5) (5, 44). In flaviviruses, E protein is involved in a number of important functions related to virus infection such as receptor binding and membrane fusion (26). Antibodies to the E protein were shown to neutralize virus activity *in vitro* as well as *in vivo* since passive administration of monoclonal antibodies to the E protein protected mice with JEV infection (17). Furthermore, sub-viral particles consisting of only the prM and the E proteins were highly effective in generating protective immune response in mice against JEV (18, 25). Chen *et al.* (7) examined the potential of various JEV structural and non-structural proteins for vaccine development. They concluded that the E protein was the single most important protein capable of inducing protective immunity against JEV. Accordingly, several plasmid vectors have been described synthesizing different forms of JEV E protein with or without prM protein and these provided protection of varying degree in mice against Japanese encephalitis (2,6, 15,20).

In recent years, adenoviruses have shown great promise as vectors for recombinant vaccine development (3,4, 8,13, 35,41, 42,49). Besides being safe, these viruses have been shown to induce effective humoral and cellular immune responses in experimental animals when delivered orally, intra-muscular(IM), intra-peritoneal or intra-nasal (3,21, 24,40, 41,50). US Patent application 5,494,671 discloses a vaccine against JEV comprising a recombinant virus expressing a C-terminally truncated flavivirus envelope protein. Jaiswal Smita et al., Journal of Virologu, vol. 77, no.23, December 2003, pages 12907-12913 discloses a vaccine against dengue virus type 2.

In the present invention, we constructed RAdEa expressing the prM and the full length JEV E protein since it is known to induce neutralizing immune response. However, adenovirus recombinant (pAdEa) synthesizing the full-length protein (Ea) did not grow well. Besides it induced poor immune response and very little JEV neutralizing antibodies, The full-length E protein is membrane anchored and removing the anchor signal from it is likely to make it soluble and perhaps more immunogenic.

On this premise, we truncated the Ea protein to remove 102-amino acid hydrophobic sequence from the C-terminus of the protein to generate a 398-amino acid Es protein.

Recombinant adenovirus RAdEs synthesizing JEV prM and Es was found to grow very well in cultured cells and synthesize JEV E protein that was secreted into the medium,

So, the removal of 102-amino acid hydrophobic sequence from the C-terminus of the protein played a critical role in smooth culturing of the recombinant. Even the inventors were pleasantly surprised with this effect.

Further, this recombinant adenovirus, synthesizing a novel form of JEV E protein that was secretory as opposed to the anchored protein in the normal course, was highly immunogenic in mice. RAdEs induced high titers of JEV neutralizing antibodies and protected the immunized mice against lethal JEV challenge demonstrating its potential use as a vaccine against JEV infection. The highly immunogenic effect of the recombinant helped achieve the desired results, which were long awaited by the scientific community.

The infection caused by JEV is fatal in nature and absence of long-term immunity had added to the problem. The allergic reactions to the known vaccine had further In the present invention, we constructed RAdEa expressing the prM and the full length JEV E protein since it is known to induce neutralizing immune response. However, adenovirus recombinant (RAdEa) synthesizing the full-length protein (Ea) did not grow well. Besides it induced poor immune response and very little JEV neutralizing antibodies. The full-length E protein is membrane anchored and removing the anchor signal from it is likely to make it soluble and perhaps more immunogenic.

On this premise, we truncated the Ea protein to remove 102-amino acid hydrophobic sequence from the C-terminus of the protein to generate a 398-amino acid Es protein.

Recombinant adenovirus RAdEs synthesizing JEV prM and Es was found to grow very well in cultured cells and synthesize JEV E protein that was secreted into the medium.

So, the removal of 102-amino acid hydrophobic sequence from the C-terminus of the protein played a critical role in smooth culturing of the recombinant. Even the inventors were pleasantly surprised with this effect.

Further, this recombinant adenovirus, synthesizing a novel form of JEV E protein that was secretory as opposed to the anchored protein in the normal course, was highly immunogenic in mice. Trades induced high titers of JEV neutralizing antibodies and protected the immunized mice against lethal JEV challenge demonstrating its potential use as a vaccine against JEV infection. The highly immunogenic effect of the recombinant helped achieve the desired results, which were long awaited by the scientific community.

The infection caused by JEV is fatal in nature and absence of long-term immunity had added to the problem. The allergic reactions to the known vaccine had further compounded the problem. However, administeration of the vaccine of instant invention is been found to be absolutely safe and free from adverse effects.

### Objects of the invention

The main object of the present invention is to develop a recombinant adenovirus (RAdEs) vaccine against JEV infection.

Another main object of the present invention is to develop an effective and superior method of immunization to Japanese encephalitis virus (JEV) infection.

Yet another object of the present invention is to develop a plasmid pAdEs of SEQ ID No. 1.

Still another object of the present invention is to develop a method of preparing a recombinant adenovirus (RAdEs) vaccine to protect against Japanese encephalitis virus (JEV) infection.

### Summary of the invention

The present invention relates to development of a novel recombinant adenovirus (RAdEs) vaccine against Japanese encephalitis virus (JEV) infection.

### Detailed description of the invention

Accordingly, the present invention relates to a novel recombinant adenovirus (trades) vaccine against JEV infection; an effective and superior method of immunization to Japanese encephalitis virus (JEV) infection; also, a plasmid pAdEs of SEQ ID No. 1; and lastly, a method of preparing the recombinant adenovirus (RAdEs) vaccine.

In the main embodiment of the present invention, it relates to a method of preparing a recombinant adenovirus (RAdEs) vaccine to protect against Japanese encephalitis virus (JEV) infection, wherein the said vaccine produces secretory envelop protein of JEV, said method comprising steps of:
- digesting plasmid pMEs with restriction enzymes *Kpn* I and *Bam* HI to obtain cDNA encoding JEV proteins prM and Es,
- ligating the cDNA to adenovirus shuttle plasmid pShuttle digested with restriction enzymes *Kpn* I and *Hind* III at the *Kpn* I end,
- filling nucleotides at the free *Bant* HI and *Hind* III ends with T4 DNA polymerase to create blunt ends,
- ligating the blunt ends together to yield shuttle plasmid pSEs with JEV cDNA encoding the proteins prM and Es,
- digesting the shuttle plasmid pSEs with restriction enzymes I-*Ceu* I and P1*-Sce* I to obtain expression cassette containing the JEV cDNA together with the CMV promoter/enhancer and BGH polyadenylation signal,
- ligating the digested shuttle plasmid with I-*Ceu* I and P1-*Sce* I digested adenovirus plasmid pAdeno-X to generate plasmid pAdEs containing the Es expression cassette,
- digesting the plasmid pAdEs with *Pac* I,
- transfecting the monolayers of HEK 293 cells with digested plasmid pAdEs for about one week, and
- obtaining the recombinant virus RAdEs vaccine.

In another embodiment of the present invention, the transfection is at about 37°C temperature.

In yet another embodiment of the present invention, the secretory proteins are under the control of human CMV IE promoter/enhancer.

In another main embodiment of the present invention, the invention relates to a recombinant adenovirus (RAdEs) vaccine.

In yet another embodiment of the present invention, the vaccine produces secretory envelop protein (Es) of JEV.

In still another embodiment of the present invention, the vaccine protects against Japanese encephalitis virus (JEV) infection.

In still another embodiment of the present invention, the vaccine is effective by intramuscular route of administration.

In another main embodiment of the present invention, the invention relates to a plasmid pAdEs of SEQ ID No. 1.

In another main embodiment of the present invention, the invention relates to an effective and superior method of immunizing a subject in need thereof, to Japanese encephalitis virus (JEV) infection, said method comprising the step of administering a pharmaceutically effective amount of recombinant virus RAdEs vaccine optionally along with additive(s) to the subject intra-muscularly.

In yet another embodiment of the present invention, the method shows 100% efficacy. In still another embodiment of the present invention, the method helps protect subject against Japanese encephalitis.

In still another embodiment of the present invention, the subject is animal.

In still another embodiment of the present invention, the subject is a human being.

In still another embodiment of the present invention, the immunization activates both humoral and cell-mediated immune responses.

In still another embodiment of the present invention, the humoral response to the vaccine is antibody IgG1 type.

In still another embodiment of the present invention, the method leads to high amount of IFN-gamma secretion.

In still another embodiment of the present invention, the immunization leads to IL-5 secretion at moderate levels.

In still another embodiment of the present invention, increased amounts of RAdEs lead to higher immune response.

In still another embodiment,of the present invention, the method is more effective than the commercially available vaccine.

Replication-defective recombinant adenoviruses (RAds) were constructed that synthesized the pre-Membrane (prM) and envelope (E) proteins of Japanese encephalitis virus, (JEV). Recombinant virus RAdEa synthesized Ea, the membrane-anchored form of the E protein, and RAdEs synthesized Es, the secretory E protein. RAdEa replicated poorly in human embryonic kidney (HEK) 293A cells and 88-folds lower titers of the virus were obtained compared to RAdEs. RAdEa also synthesized lower amounts of E protein in HEK 293A cells as judged by radioimmunoprecipitation and immunofluorescence studies.

Mice were immunized intra-muscular (IM) and orally with RAds. Oral route of virus delivery induced low titers of anti-JEV antibodies that had only little JEV neutralizing activity. IM immunizations with both RAdEa and RAdEs resulted in high titers of anti-JEV antibodies. Interestingly, RAdEa induced very low titers of JEV neutralizing antibodies whereas RAdEs inoculation resulted in high titers of JEV neutralizing antibodies. Splenocytes from mice immunized IM with RAds secreted large amounts of interferon-γ and moderate amounts of interleukin-5. These splenocytes also showed cytotoxic activity against JEV-infected cells. Mice immunized IM with RAdEs showed complete protection against the lethal dose of JEV given intra-cerebral.

### Brief description of the accompanying drawings:

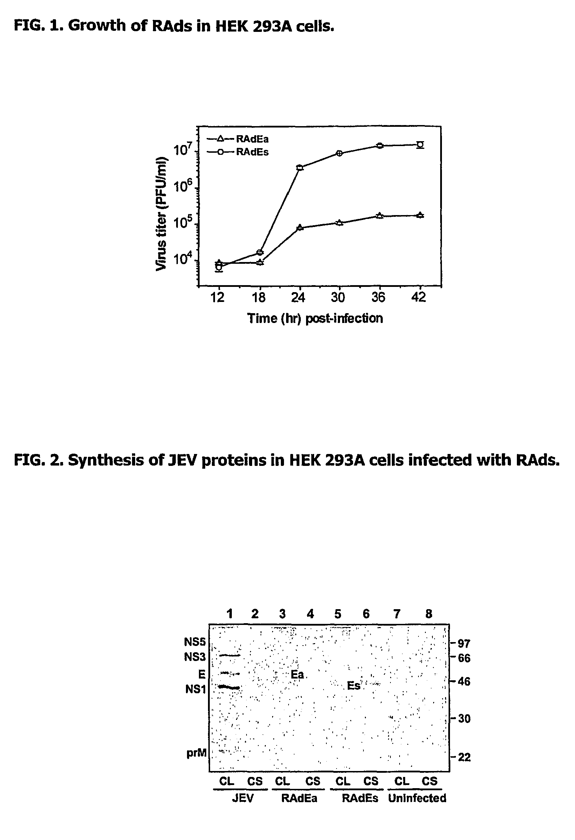

**Fig. 1** shows Growth of RAds in HEK 293A cells. Monolayers of HEK 293A cells in 35-mm tissue culture dishes were infected with RAds at a multiplicity of infection (MOI) of.1 and incubated at 37 °C in 3 ml culture medium. At 6 hr interval the cell monolayers were lysed in the culture medium by three cycles of freeze-thawing. The cell lysate was centrifuged to remove the debris and the supernatant assayed for adenovirus titers on HEK 293A cells. Shown in the figure are virus titers at various time points.
**Fig. 2** shows Synthesis of JEV proteins in HEK 293A cells infected with RAds. HEK 293A cells were infected with JEV or RAds at a MOI of 1. Cells were labelled 24 hr later by growth in medium containing ³⁵S-methionine and ³⁵S-eystine. After labeling, culture supernatant (CS) and cells were harvested separately. Cells were washed with PBS before the cell lysate (CL) was made. CLs and CSs were used for immunoprecipitation of JEV proteins with mouse anti-JEV serum. The proteins were separated on a 12% SDS-polyacrylamide gel and autoradiographed. Lanes containing proteins precipitated from JEV-, RAdEa- or RAdEs-infected or uninfected cells have been identified. Position of JEV proteins has been indicated at the left. Ea, Es and prM proteins synthesized by RAds have been indicated. The values on the right are molecular size markers in kDa.

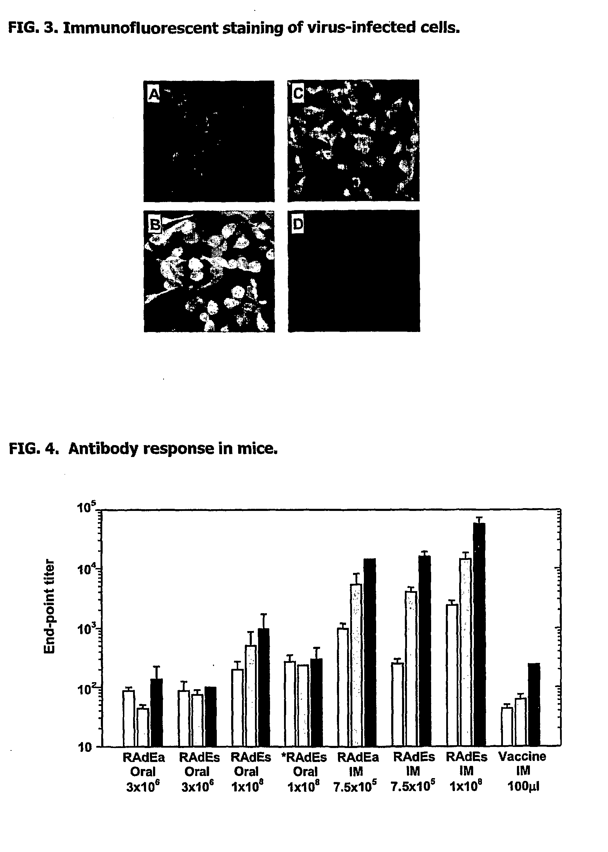

**Fig. 3** shows Immunofluorescent staining of virus-infected cells. HEK 293A cells were infected with RAds or JEV at a MOI of 1. The cells were fixed and permeabilized 24 hr later and stained with mouse anti-JEV serum followed by FITC-conjugated goat anti-mouse-IgG. They were then observed under a microscope using ultra-violet light. Panel A, RAdEa-infected cells; panel B, RAdEs-infected cells; panel C, JEV-infected cells and panel D, uninfected cells.
**Fig. 4** shows Antibody response in mice. BALB/c mice were immunized with RAdEa, RAdEs or with the vaccine by IM or oral route of inoculation. The dosages of the immunogens (in PFU for RAds) and the routes of inoculation have been indicated below the figure. *indicates oral immunization where virus was diluted in 100 mM Sodium bicarbonate buffer (pH 8.9). The primary immunization was followed by booster doses that were given 21 and 36 days later. Mice were bled at day 20, 28 and 44 post-immunization and sera stored at -70 °C. Serial two-fold dilutions of sera (starting at 1:25) were assayed for the end-point anti-JEV antibody titers by ELISA.

Shown above are mean end-point titers of sera obtained from immunized mice as indicated below the figure. The open bars represent titers on day 20, the gray bars represent titers on day 28 and black bars show titers on day 44 post-immunization.

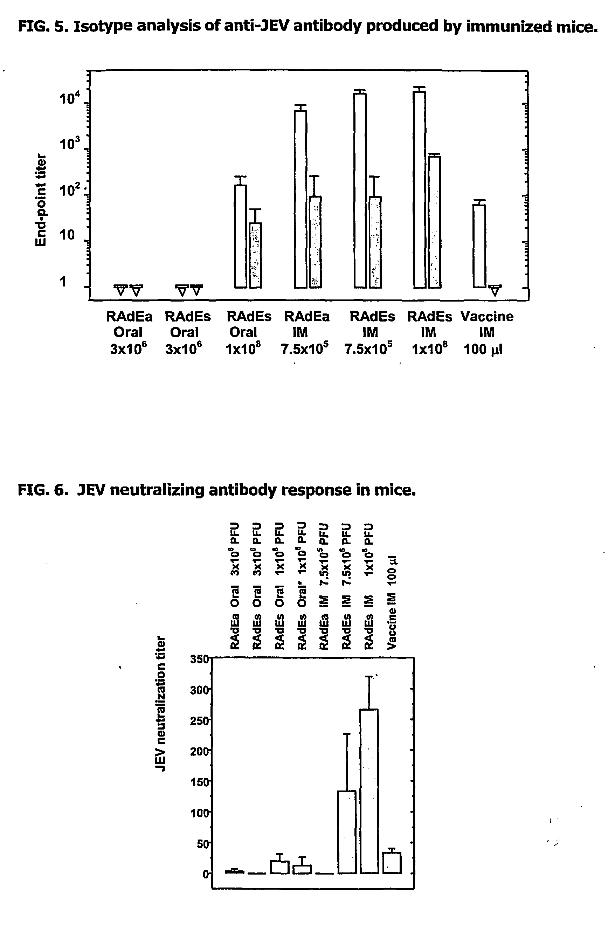

**Fig. 5** shows Isotype analysis of anti-JEV antibody produced by immunized mice. BALB/c mice were immunized with RAdEa, RAdEs or with the vaccine by IM or oral route of inoculation. The dosages of the immunogens (in PFU for RAds) and the routes of inoculation have been indicated below the figure. The primary immunization was followed by booster doses given 21 and 36 days later. Mice sera obtained on day 44 post-immunization were assayed for the end-point ELISA titers of anti-JEV IgG 1 and IgG2a antibodies. Serial two-fold dilutions of sera (starting at 1:25) were assayed for the end-point titers. The ELISA titer was recorded as zero if the 1:25 dilution of sample was negative in ELISA. Shown in the figure are mean end-point titers of sera obtained from immunized mice as indicated below the figure. The open bars represent IgG1 titers and the gray bars represent IgG2a titers. The inverted triangle indicates zero mean titer.
**Fig. 6** shows JEV neutralizing antibody response in mice. BALB/c mice were immunized with RAdEa, RAdEs or the vaccine by 1M or oral route of inoculation. The dosages of the immunogens and the route of inoculation have been indicated at the top of the figure. The primary immunization was followed by booster doses given 21 and 36 days later. *indicates oral immunization where virus was diluted in 100 mM Sodium bicarbonate buffer. Mice were bled on day 44 post-immunization and sera stored at -70°C. Serial two-fold dilutions of sera (starting at 1:10) were assayed for end-point JEV neutralization titers by plaque reduction neutralization assays. Shown in the figure are mean JEV neutralization titers of serum samples from immunized mice.

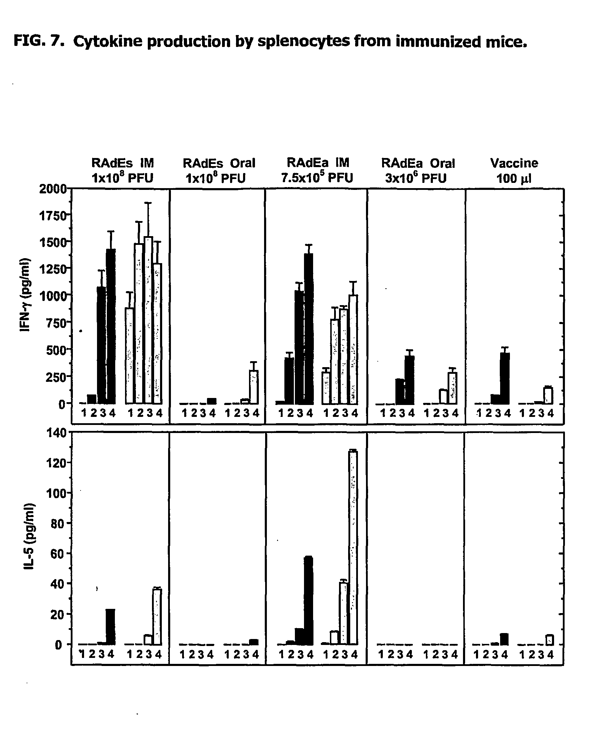

**Fig. 7** shows Cytokine production by splenocytes from immunized mice. BALB/c mice were immunized with RAdEa, RAdEs or the vaccine by IM or oral route of inoculation as indicated at the top of the figure. These mice were given two booster doses as described in the methods. One week after the second booster dose splenocytes from two mice (indicated by gray and black bars) from each immunization group were cultured in presence of JEV. Culture supernatants were collected each day and stored at -70°C. These were then assayed for IFN-γ, IL-4 and IL-5. Shown in the figure are the levels of IFN-γ and IL-5 in splenocyte cultures on various days after incubation with JEV. The days are numbered at the bottom of the panel.

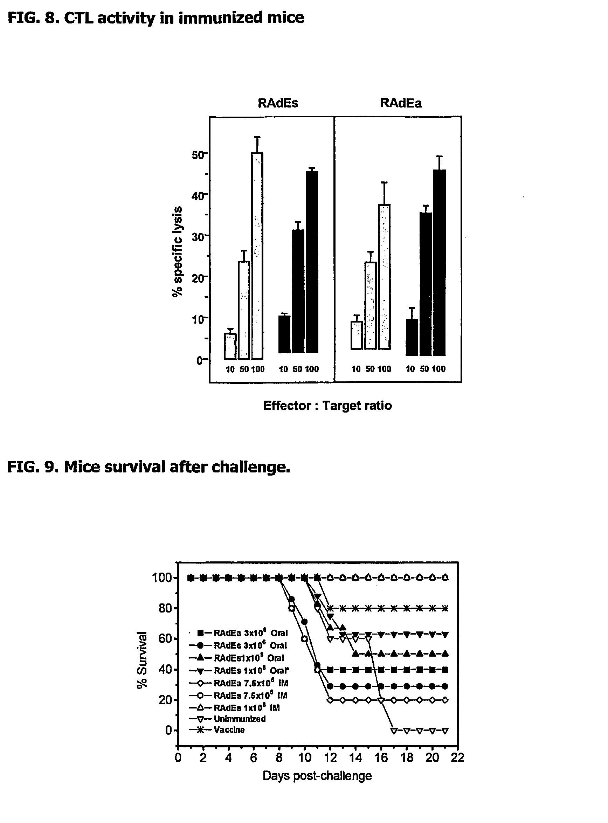

**Fig. 8** shows CTL activity in immunized mice. BALB/c mice were immunized with RAdEa or RAdEs by IM route of inoculation. These mice were given two booster doses as described in the methods. One week after the second booster dose splenocytes from two mice (indicated by gray and black bars) from each immunization group were cultured in presence of JEV for the generation of effector cells. Shown in the figure is the CTL activity of splenocytes at various ratios of effector cells to target cells (E : T) which are indicated at the bottom of the panels.
**Fig. 9** shows Mice survival after challenge. Groups of 6-8 BALB/c mice were immunized with various dosages of RAdEa and RAdEs through IM or oral route. These mice received two booster doses of the immunogen on day 22 and 36 post-immunization. Mice were challenged on day 44 post-immunization with 100 LD₅₀ (50%-lethal dose) of JEV given intra-cerebrally. Mice were observed for mortality for the next three weeks. Shown above is the percentage of surviving mice at a given time point. Immunogen dose and route of inoculation have been indicated. *indicates oral delivery of RAdEs along with the bicarbonate buffer.

The invention is further elaborated with the help of experimental data, as presented below in the form of examples. However, the examples should not be construed to limit the scope of the invention

### Example - 1

**JEV and cells:** The GP78 strain of JEV was used in these studies (44). The virus was grown in neonatal mouse brain. The brain from infected mice was homogenized as a 10%, suspension in Eagle's minimal essential medium (EMEM). The suspension was centrifuged and filtered through 0.22 µm sterile filters. The virus was stored at -70 °C in aliquots. Virus titration was carried out by plaque assay on porcine stable kidney (PS) monolayers as described previously (43). Adenovirus was grown in human embryonic kidney (HEK) 293A cells (Quantum Biotechnologies Inc.) cultured in Dulbecco's modified Eagle's medium (DMEM) supplemented with 10% fetal calf serum (FCS).

### Example - 2

**Construction of recombinant adenoviruses:** Recombinant adenoviruses were constructed using the Adeno-X expression system (BD Biosciences) that utilized a ligation-based strategy for producing recombinant virus. Using this system a mammalian expression cassette containing the cDNA encoding JEV E protein was incorporated into a replication incompetent ( E1/ E3) human adenovirus type 5 (Ad5) genome. Two recombinant adenoviruses (RAds) were made; RAdEa, synthesizing JEV prM and the membrane-anchored E protein, and RAdEs, synthesizing prM and the secretory E protein. Plasmids pMEa and pMEs that contained the cDNAs encoding JEV prM and the membrane-anchored (Ea) or secretory E protein (Es), respectively have been described previously (15). Plasmid pMEa was modified by site-directed mutagenesis to contain an *Afl* II restriction site down-stream of the 3'-end of the JEV cDNA past the *Bam* HI site. The JEV cDNA encoding the prM and Ea proteins was excised from the mutated pMEa as a *Kpn* I - *Afl* II fragment and cloned at these sites in the adenovirus shuttle plasmid pShuttle (BD Biosciences) under the control of human cytomegalovirus (CMV) immediate early (IE) promoter/enhancer to yield plasmid pSEa. The shuttle plasmid pSEa contained the bovine growth hormone (BGH) polyadenylation signal downstream of the cloned JEV cDNA. The cDNA encoding prM and Es sequence was obtained by digesting pMEs with *Kpn* I and *Bam* HI. This cDNA fragment was ligated to the adenovirus shuttle plasmid, pShuttle, digested with *Kpn* I and *Hind* III at the *Kpn* I end. The free *Bam* HI and *Hind* III ends were nucleotide filled with T4 DNA polymerase and the blunt ends so created were ligated together to yield plasmid pSEs where JEV cDNA encoding prM and Es was under the control of human CMV IE promoterlenhancer. The shuttle plasmid pSEs contained the BGH polyadenylation signal downstream of the cloned cDNA. The junctions of the shuttle plasmid and the cDNA insert were sequenced to confirm the presence of the cDNA in correct frame. The plasmids pSEa and pSEs were digested with I-*Ceu* I and PI-*Sce* I to obtain the expression cassettes containing the JEV cDNA together with CMV promoter/enhancer and BGH polyadenylation signal. These were then ligated with I-*Ceu* I and P1-*Sce* I digested adenovirus plasmid pAdeno-X (BD Biosciences) to generate plasmids pAdEa and pAdEs containing the Ea and Es expression cassettes, respectively. Monolayers of HEK 293A cells were transfected with *Pac* I digested pAdEa and pAdEs using Effectene (Qiagen) and incubated for a week at 37 °C. By this time, RAd plaques had begun to show. The cell monolayers were harvested in culture supernatant, frozen-thawed thrice and centrifuged to obtain the released crude recombinant virus that was amplified once in HEK 293A cells and subjected to 2 rounds of plaque purification to obtain recombinant viruses RAdEa and RAdEs.

### Example - 3

**Radioimmunoprecipitation:** Synthesis of JEV proteins by RAdEa and RAdEs was studied by infection of HEK 293A cells followed by radiolabelling and immunoprecipitation. Briefly, monolayers of HEK 293A cells were infected with virus at a multiplicity of infection (MOI) of 1 and incubated at 37 °C. After 24 hr, the cell monolayer was radiolabelled by growing in presence of 100 µCi of Easytag^{™} EXpre³⁵S³⁵S protein labeling mix (NEN) for 4 hr. The culture supernatant was harvested and stored at -70 °C. The monolayers were harvested in 500 µl of radioimmunoprecipitation assay buffer (10 mM Tris-HCl pH 8.0, 140 mM NaCl, 5 mM Iodoacetamide, 0.5% Triton X-100, 1% Sodium dodecyl sulphate (SDS), 1% Sodium deoxycholate, 2 mM Phenylmethylsulfonyl fluoride). Immunoprecipitation was carried out using mouse anti-JEV serum (ATCC) and Protein A Sepharose beads (Amersham). Immunoprecipitated proteins were electrophoresed on a 12% SDS-polyacrylamide gel that was dried before exposing to X-ray film for autoradiography.

### Example - 4

**Immunofluorescent staining:** Monolayers of HEK 293A cells were infected with RAdEa, RAdEs or JEV at a MOI of 1. The cells were fixed the next day with 2% Parafonnaldehyde and permeabilized with 0.1% Triton-X 100. These cells were then stained by incubation with mouse anti-JEV serum (ATCC) followed by anti-mouse IgG-FITC conjugate (Dako) and observed under a microscope using ultra-violet light.

### Example - 5

**Mice immunization and challenge experiments:** All immunizations were carried out on 4-5-week-old inbred BALB/c mice. Each immunization group consisted of 6-8 mice. For IM immunizations, mice were injected in the hind legs with different amounts of RAds diluted in 100 µl phosphate-buffered saline (PBS) using a 30G needle. Another group of mice was immunized with the mouse brain-derived, formalin-inactivated JEV vaccine manufactured by the Central Research Institute, Kasauli (India). Each mouse was given an IM injection of 100 µl of the vaccine that was 1/10^{th} of the recommended adult human dose. For oral immunizations, water was withdrawn from the mice cages for 6-8 hr and then 200-400 µl virus diluted in PBS or 100 mM Sodium bicarbonate buffer (ph 8.9) was administered per oral using a mouse-feeding needle that had a small balloon at the point of the delivery. Two booster doses, given 3 and 5 weeks after the primary immunization, contained the same amount of immunogen as the primary dose. At 44 days post-immunization, mice were challenged with intra-cerebral inoculation of the lethal dose of JEV. These mice were observed for mortality for the next 3 weeks.

### Example - 6

**Antibody assays:** Titers of anti-JEV antibodies were assayed using an enzyme-linked immunosorbent assay (ELISA). An ELISA plate was coated with C6/36 cell-grown JEV overnight at 4 °C in 0.2 M Sodium carbonate buffer, pH 9.6 (36). The plate was washed with PBS containing 0.1% Tween-20 (PBS-T) thrice and the wells were blocked with 1% Lactogen in PBS-T at 37 °C for 2 hr. The plate was again washed with PBS-T thrice and incubated with 100 µl diluted mice sera per well at 37 °C for 1 hr. Serial two-fold dilutions of sera were assayed starting at a dilution of 1:25. The plate was then washed thrice with PBS-T and 100-µl anti-mouse antibody conjugated to horse radish peroxidase (HRP) (Dako), diluted 1:2000, was added per well, followed by incubation at 37 °C for 1 hr. The antibody-conjugate was removed by washing the plate thrice with PBS-T. The plate was then incubated in dark with 100 µl per well of the substrate o-Phenylenediamine dihydrochloride (0.5 mg/ml) prepared in citrate buffer (1% Citric acid and 1.46% Disodium hydrogen phosphate) at room temperature for 10 min. The reaction was stopped by adding 50 µl of 5 N Sulfuric acid. The absorbance was read at 492 nm in an ELISA plate reader (Spectramax). The reciprocal of the highest serum dilution giving an optical density at least twice that given by the reagent blanks was taken as the ELISA end-point.

The antibody isotyping ELISAs were carried out in a similar fashion, except that in place of anti-mouse IgG-HRP conjugate, anti-mouse IgG1-HRP or the IgG2a-HRP conjugate (Pharmingen) was added. This was followed by the incubation with the substrate and color development as above.

### Example - 7

**Plaque reduction neutralization assay:** Two fold serial dilutions of sera from the immunized mice (starting from 1:10) were prepared in EMEM containing 5% FCS and antibiotics. Diluted sera were incubated at 56 °C for 30 min to inactivate the complement. The serum sample (100 µl) was then mixed with equal volume of JEV culture supernatant containing 100 plaque-forming units (PFU) of the virus. The virus-antibody mixture was incubated at 37 °C for 1 hr before adding to a 35-mm dish containing ∼70% confluent monolayer of PS cells. The plaque assay was carried out as described. (43). Percent neutralization was calculated by counting the number of plaques in the presence and the absence of the mouse serum. All assays were done in duplicates. Reciprocal of the highest serum dilution giving 50% neutralization was taken as the JEV neutralization titer.

### Example - 8

**Cytokine ELISA:** Spleen cell suspensions were prepared in RPMI 1640 supplemented with 10% FCS and 6x10⁷ splenocytes were incubated with 3x10⁷ PFU of JEV or *E*. *coli*-synthesized JEV E protein (10 µg/ml) in a 35-mm dish at 37 °C. Aliquots of culture supernatant were removed every day for the next 4 days and stored at -70°C. Interleukin (IL)-4, IL-5 and interferon (IFN)-γ were assayed using BD OptEIA kits (BD Biosciences) and as per the assay protocols.

### Example - 9

**Cytotoxic T lymphocyte (CTL) assay:** Standard ⁵¹Cr-release method as described previously (15) was used for the CTL assays with some modifications. Briefly, for preparation of the responder cells, 3x10⁷ splenocytes were incubated for 4 days with 3x10⁷ PFU of JEV in a 35-mm dish in RPMI 1640 medium supplemented with antibiotics, 0.5 mM β-Mercaptoethanol, 0.32 mg/ml L-Glutamine, 0.1 mg/ml non-essential amino acids, 0.12 mg/ml Sodium pyruvate and 5% FCS at 37 °C. The cells thus generated were referred to as the effector cells. Virus-infected target cells were prepared by infecting P388D1 cells with JEV for 48 hr at a MOI of 1 followed by incubation with 100 µCi of Na₂⁵¹crO₄ (NEN) and subsequent washings to remove free ⁵¹Cr.

For carrying out the CTL assay, various numbers of effector cells were incubated at 37°C for 6 hr with 2x10^{4 51}Cr-Iabelled virus-infected cells by briefly centrifuging them at 100 g for 4 min in a 96-well round bottom plate. At the end of the incubation period, 100 µl of cell-free supernatant was removed and the ⁵¹Cr release was counted using a gamma counter (LKB). Triplicate estimations were done in all the assays and the percentage lysis was calculated using the following formula. Percent lysis = [(cpm released in the presence of effector cells - cpm released due to spontaneous leakage)/(total cpm released by 0.2% Triton X-100 lysis - cpm released due to spontaneous leakage)] x 100.

**Statistical analysis:** The statistical significance of different findings between mouse groups was determined by Student's *t* test. P ≤0.05 was considered to be significant.

### Example - 10

**Replication of RAds and synthesis of JEV proteins:** The JEV E protein is 500 amino acids long membrane-anchored protein. We have earlier shown that deletion of the C-terminal 102 amino acids of JEV E protein leads to efficient secretion of the truncated protein into the cell surroundings (15). We had also shown that quality of immune responses in mice induced by the secretory E protein were different from those induced by the membrane-anchored E protein (15). Besides, vaccinia recombinants expressing the secretory E protein of Japanese encephalitis or Dengue viruses devoid of the membrane anchor sequence were found to be highly immunogenic in mice (14, 27, 37). We, therefore, constructed two recombinants, RAdEa synthesizing prM and Ea (full-length membrane-anchored JEV E protein) and RAdEs synthesizing prM and Es (398 amino acid secretory E protein). The JEV prM was included in our constructs as its co-synthesis was necessary for correct processing and folding of the E protein (18, 25). The presence of appropriate JEV cDNA in genomes of RAds was established by polymerase chain reaction using JEV genome sequence-specific oligonucleotide primers.

Replication of RAds was studied in HEK 293A cells infected at a MOI of 1. Figure 1 shows that a major burst in viral titers was observed both for RAdEa and RAdEs between 18 and 24 hr post-infection (PI) after which there was only a marginal increase in titers. RAdEs titer was ∼40-fold higher than that of RAdEa at 24 hr PI. Following the virus replication, the cytopathic effects were first visible at 30 hr PI for both RAdEa and RAdEs. The cytopathic effects had become highly pronounced by 42 hr PI when almost 80% cells had come off the surface of the tissue culture plates. At this time point titer of RAdEs was 1.2x10⁷ PFU/ml, which was 88-times higher than that of RAdEa. These results showed that compared to RAdEs, RAdEa replicated poorly in HEK 293A cells. We studied four independent isolates of RAdEa and found all them to be slow growers.

The synthesis of JEV proteins by RAds was studied in HEK 293A cells that were infected with different viruses followed by radiolabelling and immunoprecipitation of JEV proteins using mouse anti-JEV serum. Figure 2 shows that RAdEa-infected cell lysate had 2 proteins of apparent molecular masses of 51 and 23 kDa that corresponded with JEV E and prM proteins. None of these proteins were detectable in the culture supernatant of the infected cells. The RAdEs-infected cell lysate showed the synthesis of Es and prM proteins of apparent molecular masses of 45 and 23 kDa, respectively. The culture supernatant from the RAdEs-infected cells had a significant amount of Es, indicating that the E protein synthesized by RAdEs was secretory.

Use of increased amounts of anti-JEV antibody and Protein A Sepharose in immunoprecipitations did not result in precipitation of enhanced amounts of E protein indicating that the amounts of these reagents used were not limiting. Scanning of the autoradiograph with optically-enhanced densitometer using 'Diversity One' software (version 1.6, PDI, New York) followed by calculations based on the volumes of the lysate or the supernatant loaded on the gel, suggested that the secretory E protein present in the culture supernatant constituted about 76% of the total E protein synthesized by RAdEs-infected cells. Furthermore, levels of total Es protein synthesis were around 20-fold higher than the levels of Ea protein. Immunofluorescent staining of RAdEa- and RAdEs-infected HEK 293A cells further confirmed that levels of Ea protein were significantly lower than that of the Es protein (Figure 3). The lower levels of Ea synthesis may be related to the poor growth of RAdEa in HEK 293A cells.

We found that RAdEa synthesizing the membrane-anchored E protein grew slowly and achieved 88-fold lower titers in HEK 293A cells when compared with RAdEs synthesizing the secretory E protein. Infection of HEK 293A cells with the recombinants followed by radioimmunoprecipitation of JEV proteins showed that RAdEa synthesized lower amounts of JEV E protein. This was corroborated by the low levels of immunofluorescence on RAdEa-infected HEK 293A cells when compared with RAdEs-infected cells. HEK 293A cells support replication of E1-deleted RAds reported in this work as these cells contain Ad5 E1 transcription unit permanently integrated in them. In other mammalian cells these RAds are unable to replicate for the want of the E1 sequences. However, expression of the foreign gene does take place, which is under the independent control of the CMV IE promoter. We have studied expression of JEV E protein by RAds in PS cells by immunofluorescence and radioimmunoprecipitation. No significant differences were found in the levels of E protein synthesis in PS cells infected with RAdEa or RAdEs at a MOI of 1.

The adenovirus vector used in our studies had deletions in both the E1 and E3 transcription units that allow packaging of up to 8 Kb foreign DNA in the recombinant virus. The size of JEV expression cassette inserted in RAdEa was ∼3.3 Kb which was well within the packaging limits of the recombinant virus. The reason for lower titers of RAdEa in HEK 293A cells is, thus, not clear. Previously, we found no differences in the levels of expression of membrane-anchored or secretory E protein of JEV when HEK 293A cells were transfected with expression plasmids containing the Ea or Es genes under CMV IE promoter (15). This observation together with our results in the PS cells on E protein synthesis indicates that lower levels of E protein synthesis by RAdEa in HEK 293A cells are related to the low levels of its replication.

### Example - 11

**Anti-JEV antibody response in mice immunized with RAds:** Groups of BALB/c mice were immunized with RAdEa or RAdEs delivered orally or IM. The antibody response of these mice was compared with those immunized with formalin-inactivated commercial JEV vaccine. Serum samples collected from mice at various time points were assayed for anti-JEV end-point ELISA titers. Figure 4 shows that anti-JEV antibodies were detectable in all immunization groups on day 20 post-immunization and these titers increased further on days 28 and 44 post-immunization following the booster doses. Compared to IM immunization, antibody titers were drastically low in mice immunized orally with RAds. For example, compared to oral immunization, 1x10⁸ PFU of RAdEs given IM induced ∼60-fold higher antibody response on day 44. For both the oral as well as IM immunizations, higher antibody titers were obtained when higher doses of RAd were used. No significant differences were seen in day 44 anti-JEV antibody titers of mice immunized IM with 7.5x10⁵ PFU of RAdEa or RAdEs. IM immunizations with RAds induced significantly higher antibody titers than those induced by IM inoculation of the vaccine. Thus, the lower dose of RAdEa and RAdEs (7.5x10⁵ PFU) gave ∼ 60-fold higher antibody titers after the second booster when compared with the titers obtained with the vaccine. At the higher dose of 1x10⁸ PFU, RAdEs induced ∼250-fold higher titers than the vaccine on day 44.

Some investigators have carried out oral immunization of mice with RAds using Sodium bicarbonate buffer to neutralize the acid pH of the stomach (11, 40, 47). However, there are others who have carried out oral immunization of mice with RAds without the use of any buffer (3, 9, 42, 48). We have compared the immunogenicity in mice of RAdEs given orally (1x10⁸ PFU) with or without the bicarbonate buffer. Figure 4 shows that no advantage was offered by the use of bicarbonate buffer during oral immunization with RAdEs. In fact, no effect of booster doses was seen when RAdEs was delivered orally using bicarbonate buffer and antibody titers on day 44 post-immunization were lower than in those mice that received the virus without bicarbonate buffer.

### Example - 12

**Isotype analyses of anti-JEV antibody produced by immunized mice:** In order to analyze the quality of immune responses generated by the RAds, end-point titers of anti-JEV IgG1 and IgG2a antibodies were determined by ELISA. Figure 5 shows that at lower dose of RAds given orally (3x10⁶ PFU) titers of anti-JEV IgG1 and IgG2a antibodies were below 25. When a higher dose of RAdEs (1x10⁸ PFU) was given orally, the majority of antibodies were of IgG1 type; the ratio of IgG1/IgG2a titers in this case was 6.48 indicating a Th2 type of immune response. When RAds were delivered through IM route, the anti-JEV antibody response was almost exclusively of IgG1 type. In case of mice immunized IM with RAdEa (7.5x10⁵ PFU), IgG1/IgG2a titer ratio was 68 and it was 64 in the case of RAdEs (7.5x10⁵ PFU)-immunized mice. At higher dose of 1x10⁸ PFU, RAdEs again induced predominantly IgG1 type of anti-JEV antibodies; the ratio of IgG1/IgG2a titers in this case was 25. These results indicated that RAdEa and RAdEs induced an almost exclusive Th2 kind of immune response in mice when delivered IM. The IM immunization of mice with the vaccine also induced IgG1 dominated antibody response indicating a Th2 type immune response.

### Example - 13

**JEV neutralizing antibody response in mice immunized with Rads:** Titers of JEV neutralizing antibodies were determined in serum samples obtained from the immunized mice at day 44 post-immunization. Figure 6 shows that oral route of immunization induced very low JEV neutralizing antibodies. Similar to ELISA titers, the JEV neutralizing antibody titers were lower in mice immunized orally with RAdEs plus the bicarbonate buffer compared to titers in those mice immunized with RAdEs without the bicarbonate buffer, although the difference was statistically insignificant. Compared to RAdEa, RAdEs given IM induced higher JEV neutralizing titers and these were enhanced further when higher dose of virus was used for immunization. IM immunization with RAdEs induced significantly higher JEV neutralizing antibody titers than those induced by the vaccine.

### Example - 14

**Cytokine secretion by splenocytes from immunized mice:** Splenocytes prepared from the immunized mice were cultured in presence of JEV and synthesis of IFN-γ, IL-4 and IL-5 was studied on each day for the next 4 days. Figure 7 shows that splenocytes from mice immunize with RAdEa or RAdEs by IM route secreted large amounts of IFN-γ. Splenocytes from mice immunized with the recombinant viruses through oral route made only small amounts of IFN-γ. Similarly, mice immunized with the vaccine made only small amounts of IFN-γ. Splenocytes from IM-immunized mice also made moderate amounts of IL-5 which was almost absent in the case of orally immunized mice or mice immunized with the vaccine. IL-4 was not detectable in any of the cases; the detection limit of IL-4 ELISA was 7.8 pg/ml. Similar pattern of cytokine secretion was observed when splenocytes were cultured in presence of JEV E protein

### Example - 15

**CTL activity in immunized mice:** To study the generation of memory CTLs, splenocytes from immunized mice were stimulated *in vitro* with JEV and examined for cytotoxic activity against cells infected with JEV. Figure 8 shows the results of CTL assays at various effector to target cell ratios. Thus, mice immunized with RAdEa or RAdEs through IM route showed significant CTL activity. No CTL activity was detectable in mice immunized with RAdEa or RAdEs through oral route. Similarly, unimmunized and Ad5-immunized mice or those immunized with the vaccine showed no CTL activity.

### Example - 16

**Mice challenge studies:** Mice immunized with RAds were challenged at day 44 post-immunization by intra-cerebral inoculation of a lethal dose (100 LD₅₀) of JEV. These mice were observed for mortality for 3 weeks after the challenge. All mice immunized with 7.5x10⁵ or 1x10⁸ PFU of RAdEs given IM survived the challenge while none of the unimmunized mice survived. Furthermore, none of the mice immunized IM or orally with 1x10⁸ PFU of E1/ E3 Ad5 survived the challenge. About 50-60% protection was seen in mice immunized with 1x10⁸ PFU of RAdEs given orally. The level of protection was lower (30%) when mice were immunized orally with lower doses of RAdEs (3x10⁵ PFU). The level of protection afforded by RAdEa immunization was very low; about 40% mice survived from those immunized with 7.5x10⁵ PFU given IM and only about 20% mice survived from the group immunized with 3x10⁶ PFU given orally. In a separate experiment, 15 mice were immunized with 1x10⁸ PFU of RAdEs and given 2 booster doses on day 21 and 35. When challenged on day 44 post-immunization with 1000 LD₅₀ of JEV, given intra-cerebral, 100% of the immunized mice survived.

The flavivirus E protein has been the antigen of choice for vaccine development using modem methods of vaccinology such as the DNA vaccination or the use of recombinant virus for antigen delivery (10, 12, 16, 28-31). This has been so because E protein plays an important role in a number of processes, including viral attachment, membrane fusion and entry into the host cell (26). Besides, flavivirus E protein induces virus-neutralizing antibodies and CTLs (14, 14, 15, 22, 23, 34). It has been shown that protection against JEV is mainly antibody dependent, and virus-neutralizing antibodies alone are sufficient to impart protection (19, 32). This was also implied from our previous observation that the formalin-inactivated JEV vaccine, which did not induce CTLs, provided protection to vaccinees against JEV (15). Thus, with a view to develop a recombinant virus-based JEV vaccine, we have constructed RAds synthesizing JEV E protein.

During replication of JEV, the E protein is expressed on the cell surface. Plasmid DNA vectors have been described that synthesize different forms of the E protein, such as the cytoplasmic, membrane-anchored or secretory (2, 6, 7, 15, 20). These different forms of JEV E protein were shown to induce different kind of immune responses. Similarly, recombinants of vaccinia expressing the secretory E protein of Japanese encephalitis or Dengue viruses were found to be highly immunogenic in mice (14, 27, 37). Previously, we had shown that truncated JEV E protein, where the membrane-anchor sequence had been removed by deletion of the C-terminal 102 amino acids, was actively secreted in the cell surroundings (15). We have now constructed RAds that synthesize the membrane-anchored or the secretory E protein.

Oral delivery of RAd has been shown to induce humoral and cellular immune responses to the protein encoded by the transgene, however, these responses have usually been weaker compared to those induced by the IM or intra-peritoneal delivery of RAd (21, 35, 42). In the present study too, oral immunization of mice by RAds resulted in significantly lower anti-JEV antibody titers when compared with the IM route of RAd delivery. Thus, mice immunized IM with 1x10⁸ PFU of RAdEs gave ∼60-fold higher anti-JEV antibody response than those immunized with the same dose of the virus given orally.

Use of Sodium carbonate buffer to neutralize the pH of the stomach made no perceptible difference to antibody titers when compared with the antibody titers induced by RAdEs given orally without the bicarbonate buffer. In fact, when bicarbonate buffer was used during oral immunization, the booster doses of RAdEs failed to enhance the anti-JEV antibody titers. For RAdEa too, oral delivery resulted in weaker anti-JEV antibody responses compared to the IM delivery of the recombinant. Thus, compared to oral delivery, IM inoculation of 7.5x10⁵ PFU of RAdEa (which was half the dose given orally) induced ∼100-fold higher anti-JEV antibody titers. The antibody titers were dose dependent. Thus higher doses of RAdEs (1x10⁸ PFU) delivered orally induced higher anti-JEV antibody titers. These titers were ∼4-fold higher than those induced by the commercial vaccine given IM. Importantly, IM inoculation of RAdEa or RAdEs at both the doses tested (7.5x10⁵ and 1x10⁸ PFU) resulted in significantly higher antibody responses than those given by the vaccine; 1x10⁸ PFU of RAdEs given IM induced ∼250-fold higher titer than the vaccine. Similar pattern was reflected in JEV neutralizing antibody titers when oral route of RAds delivery was compared with the IM route although the differences weren't so pronounced. Thus ∼20-fold higher JEV neutralizing antibody titers were induced by IM inoculation of 1x10⁸ PFU of RAdEs compared to oral delivery of the recombinant. Only statistically insignificant differences were noted in anti-JEV antibody titers induced by 7.5x10⁵ PFU of RAdEa and RAdEs given IM. However, there was significant difference, in the JEV neutralizing antibody titers generated by the two RAds; a mean JEV neutralizing antibody titer of 10 was obtained when mice were immunized with RAdEa whereas it was 133 when mice were immunized with RAdEs. Higher JEV neutralizing antibody titers were recorded when higher doses of RAdEs were used for immunization. At both the doses tested (7.5x10⁵ and 1x10⁸ PFU) IM inoculation of RAdEs induced significantly higher JEV neutralizing antibody titers than the commercial vaccine. Thus a dose of 1x10⁸ PFU of RAdEs given IM induced 8-fold higher JEV neutralization titer than the vaccine. These differences in JEV neutralizing antibody titers induced by RAdEa and RAdEs by oral and IM inoculation were reflected in the level of protection afforded by these recombinants to the immunized mice against lethal JEV challenge. Thus both doses of RAdEs inducing JEV neutralizing antibody titers higher than the vaccine gave 100% protection. Immunization with RAdEs or RAdEa given orally gave only low levels of protection. Challenge experiments indicated that mice immunized IM with the adenovirus synthesizing the membrane-anchored form of JEV E protein did not develop protective immunity whereas those immunized with recombinant synthesizing the secretory form of JEV E protein developed robust anti-JEV protective immunity resulting in 100% protection. These results show that RAd-based JEV immunizations are superior to naked DNA immunizations, which imparted only about 50-60% protection in a mouse challenge model (15). It is interesting that level of protection was similar (50-60%) when mice were immunized with plasmid DNA synthesizing Ea or Es proteins (15) whereas in the present work Es induced significantly superior protective immune response than the Ea protein. This may be related to a more efficient delivery of JEV transgene using RAd than the direct injection of naked plasmid DNA for immunization. Our finding is, however, consistent with reports from others where truncated form of JEV or Dengue E protein (leading to its secretion) was found to be more immunogenic than the membrane-anchored form of the E protein (14, 37).

Poor anti-JEV antibody induction by oral immunization with RAds was also reflected in poor cytokine secretion by splenocytes in presence of JEV. While very little IFN-γ was secreted by splenocytes from mice immunized orally with RAdEs or RAdEa, significant amounts of IFN-γ were secreted by splenocytes obtained from mice immunized IM with RAdEa or RAdEs. Splenocytes from IM immunized mice also synthesized moderate amounts of IL-5 that was not detectable in cultures of splenocytes obtained from mice immunized orally with RAds. Mice immunized IM with both RAdEa and RAdEs had significant CTL activity, which was undetectable in mice immunized orally with RAds or IM with the vaccine. Oral immunization with RAdEs at lower dose resulted in IgG1 dominated immune responses; ratio of IgG1/IgG2a end-point titers was 6.5. This is consistent with studies on oral immunization of mice with RAd synthesizing rabies glycoprotein where abundance of anti rabies IgG1 was recorded (46). The IM inoculation of RAdEa and RAdEs also resulted in preponderance of IgG1 kind of antibodies. No data could be found in literature on antibody isotypes when adenovirus recombinants are delivered IM. Our results indicating the preponderance of IgG1 type antibodies, secretion of IFN-γ and IL-5 by splenocytes and induction of CTLs suggest that IM inoculation of mice with RAds synthesizing JEV E protein activates both the humoral and the cellular arms of the immune system, and immune responses of both Th1 and Th2 type are induced.

The results presented in this invention show that RAd synthesizing the secretory form of JEV E protein imparted robust immunity in mice against lethal dose of JEV given intra-cerebral. This makes RAdEs a potential candidate vaccine against JEV. Further, safety profile of the vaccine of the instant application was studied. The vaccine is found to be safe for administration. None of the immunized mice showed any obvious complications.

### Application Project

*<120> Title: A recombinant vaccine against Japanese encephalitis virus (JEV) infection and a method ther eof*
*<130> AppFileReference : IP 1340*
*<140> CurrentAppNumber : 1516*/*Del*/*203*
   *<141> CurrentFilingDate : 2003-12-04*

### Sequence

*<213> OrganismName : Artificial Sequence*
*<400> PreSequenceString:*

cgtaactata acggtcctaa ggtagcgaaa gctcagatct ggatctcccg atcccctatg 60
gtcgactctc agtacaatct gctctgatgc cgcatagtta agccagtatc tgctccctgc 120
ttgtgtgttg gaggtcgctg agtagtgcgc gagcaaaatt taagctacaa caaggcaagg 180
cttgaccgac aattgcatga agaatctgct tagggttagg cgttttgcgc tgcttcgcga 240
tgtacgggcc agatatacgc gttgacattg attattgact agttattaat agtaatcaat 300
tacggggtca ttagttcata gcccatatat ggagttccgc gttacataac ttacggtaaa 360
tggcccgcct ggctgaccgc ccaacgaccc ccgcccattg acgtcaataa tgacgtatgt 420
tcccatagta acgccaatag ggactttcca ttgacgtcaa tgggtggact atttacggta 480
aactgcccac ttggcagtac atcaagtgta tcatatgcca agtacgcccc ctattgacgt 540
caatgacggt aaatggcccg cctggcatta tgcccagtac atgaccttat gggactttcc 600
tacttggcag tacatctacg tattagtcat cgctattacc atggtgatgc ggttttggca 660
gtacatcaat gggcgtggat agcggtttga ctcacgggga tttccaagtc tccaccccat 720
tgacgtcaat gggagtttgt tttggcacca aaatcaacgg gactttccaa aatgtcgtaa 780
caactccgcc ccattgacgc aaatgggcgg taggcgtgta cggtgggagg tctatataag 840
cagagctctc tggctaacta gagaacccac tgcttactgg cttatcgaaa ttaatacgac 900
tcactatagg gagacccaag ctggctagcg tttaaacggg ccctctagac tcgagcggcc 960
gccactgtgc tggatgatcc gagctcggta ccatgtggct cgcaagcttg gcagttgtca 1020
tagcttgcgc aggagccatg aagttgtcga atttccaggg gaagcttttg atgaccatca 1080
acaacacgga cattgcagac gttatcgtga ttcccacctc aaaaggagag aacagatgct 1140
gggtccgggc aatcgacgtc ggttacatgt gtgaggacac catcacgtac gaatgtccta 1200
agcttactgc gggcaatgat ccagaggacg tggattgctg gtgtgacaac caagaagtct 1260
acgtccaata tggacggtgc acgcggacca ggcattccaa gcgaagcagg agatccgtgt 1320
cggtccagac acatggggag agttcactag tgaataaaaa agaggcttgg ctggattcaa 1380
cgaaagccac acgatatctc acgaaaactg agaactggat cataaggaat cctggctatg 1440
ctttcttggc ggcggtactt ggctggatgc ttggcagtaa caacggtcaa cgcgtggtat 1500
ttaccatcct cctgctgttg gtcgctccgg cttacagttt taattgtctg ggaatgggca 1560
atcgcgactt catagaagga gccagtggag ccacttgggt ggacttggtg ctagaaggag 1620
atagctgttt gacaatcatg gcaaacgata aaccaacatt ggatgtccgc atgattaaca 1680
tcgaagctag ccaacttgct gaggtcagaa gttattgcta tcatgcctca gtcactgaca 1740
tctcgacggt ggctcggtgc cccatgactg gagaagctca caacgagaag cgagctgata 1800
gtagctatgt gtgcaaacaa ggcttcactg atcgtgggtg gggcaacgga tgtggacttt 1860
tcgggaaggg aagcattgac acatgtgcaa aattctcctg caccagcaaa gcgattggga 1920
gaacaatcca gccagaaaac atcaaatacg aagttggcat ttttgtgcat ggaaccacca 1980
cttcggaaaa ccatgggaat tactcagcgc aagttggggc gtcccaggcg gcaaagttta 2040
ccgtaacacc caatgctcct tcgataaccc tcaaacttgg tgactacgga gaagtcacac 2100
tggactgtga gccaaggagt ggactgaaca ctgaagcgtt ttacgtcatg accgtggggt 2160
caaagtcatt tctggtccat agggaatggt ttcatgacct cgctctcccc tggacgtccc 2220
cttcgagcac agcgtggaga aacagagaac tcctcatgga atttgaagag gcgcacgcca 2280

caaaacagtc cgttgttgct cttggttcac aggaaggagg cctccatcag gcgttggcag 2340
gagccatcgt ggtggagtac tcaagctcag tgaagttaac atcaggccac ctgaaatgta 2400
ggctgaaaat ggacaaacta gctctgaaag gcacaaccta tggcatgtgt acagaaaaat 2460
tctcgttcgc gaaaaatccg gcggacactg gtcacggaac agttgtcatt gaactctcct 2520
actctgggag tgatggcccc tgcaaaattc cgattgtctc cgtcgcgagc ctcaatgaca 2580
tgactcctgt tgggcggctg gtgacagtga acccctttgt cgcggcttcc agtgccaact 2640
caaaggtgct ggtcgagatg gaacccccct tcggagactc ctatatcgtg gttggaaggg 2700
gagacaagca gatcaaccac cattggcaca aataataaag gatctgttgt ttgcccctcc 2760
cccgtgcctt ccttgaccct ggaaggtgcc actcccactg tcctttccta ataaaatgag 2820
gaaattgcat cgcattgtct gagtaggtgt cattctattc tggggggtgg ggtggggcag 2880
gacagcaagg gggaggattg ggaagacaat agcaggcatg ctggggatgc ggtgggctct 2940
atggcttctg aggcggaaag aaccagcaga tctgcagatc tgaattcatc tatgtcgggt 3000
gcggagaaag aggtaatgaa atggcatcga ctcgaagatc tgggcgtggt taagggtggg 3060
aaagaatata taaggtgggg gtcttatgta gttttgtatc tgttttgcag cagccgccgc 3120
cgccatgagc accaactcgt ttgatggaag cattgtgagc tcatatttga caacgcgcat 3180
gcccccatgg gccggggtgc gtcagaatgt gatgggctcc agcattgatg gtcgccccgt 3240
cctgcccgca aactctacta ccttgaccta cgagaccgtg tctggaacgc cgttggagac 3300
tgcagcctcc gccgccgctt cagccgctgc agccaccgcc cgcgggattg tgactgactt 3360
tgctttcctg agcccgcttg caagcagtgc agcttcccgt tcatccgccc gcgatgacaa 3420
gttgacggct cttttggcac aattggattc tttgacccgg gaacttaatg tcgtttctca 3480
gcagctgttg gatctgcgcc agcaggtttc tgccctgaag gcttcctccc ctcccaatgc 3540
ggtttaaaac ataaataaaa aaccagactc tgtttggatt tggatcaagc aagtgtcttg 3600
ctgtctttat ttaggggttt tgcgcgcgcg gtaggcccgg gaccagcggt ctcggtcgtt 3660
gagggtcctg tgtatttttt ccaggacgtg gtaaaggtga ctctggatgt tcagatacat 3720
gggcataagc ccgtctctgg ggtggaggta gcaccactgc agagcttcat gctgcggggt 3780
ggtgttgtag atgatccagt cgtagcagga gcgctgggcg tggtgcctaa aaatgtcttt 3840
cagtagcaag ctgattgcca ggggcaggcc cttggtgtaa gtgtttacaa agcggttaag 3900
ctgggatggg tgcatacgtg gggatatgag atgcatcttg gactgtattt ttaggttggc 3960
tatgttccca gccatatccc tccggggatt catgttgtgc agaaccacca gcacagtgta 4020
tccggtgcac ttgggaaatt tgtcatgtag cttagaagga aatgcgtgga agaacttgga 4080
gacgcccttg tgacctccaa gattttccat gcattcgtcc ataatgatgg caatgggccc 4140
acgggcggcg gcctgggcga agatatttct gggatcacta acgtcatagt tgtgttccag 4200
gatgagatcg tcataggcca tttttacaaa gcgcgggcgg agggtgccag actgcggtat 4260
aatggttcca tccggcccag gggcgtagtt accctcacag atttgcattt cccacgcttt 4320
gagttcagat ggggggatca tgtctacctg cggggcgatg aagaaaacgg tttccggggt 4380
aggggagatc agctgggaag aaagcaggtt cctgagcagc tgcgacttac cgcagccggt 4440
gggcccgtaa atcacaccta ttaccggctg caactggtag ttaagagagc tgcagctgcc 4500
gtcatccctg agcagggggg ccacttcgtt aagcatgtcc ctgactcgca tgttttccct 4560
gaccaaatcc gccagaaggc gctcgccgcc cagcgatagc agttcttgca aggaagcaaa 4620
gtttttcaac ggtttgagac cgtccgccgt aggcatgctt ttgagcgttt gaccaagcag 4680
ttccaggcgg tcccacagct cggtcacctg ctctacggca tctcgatcca gcatatctcc 4740
tcgtttcgcg ggttggggcg gctttcgctg tacggcagta gtcggtgctc gtccagacgg 4800
gccagggtca tgtctttcca cgggcgcagg gtcctcgtca gcgtagtctg ggtcacggtg 4860
aaggggtgcg ctccgggctg cgcgctggcc agggtgcgct tgaggctggt cctgctggtg 4920
ctgaagcgct gccggtcttc gccctgcgcg tcggccaggt agcatttgac catggtgtca 4980
tagtccagcc cctccgcggc gtggcccttg gcgcgcagct tgcccttgga ggaggcgccg 5040
cacgaggggc agtgcagact tttgagggcg tagagcttgg gcgcgagaaa taccgattcc 5100
ggggagtagg catccgcgcc gcaggccccg cagacggtct cgcattccac gagccaggtg 5160
agctctggcc gttcggggtc aaaaaccagg tttcccccat gctttttgat gcgtttctta 5220
cctctggttt ccatgagccg gtgtccacgc tcggtgacga aaaggctgtc cgtgtccccg 5280

tatacagact tgagaggcct gtcctcgagc ggtgttccgc ggtcctcctc gtatagaaac 5340
tcggaccact ctgagacaaa ggctcgcgtc caggccagca cgaaggaggc taagtgggag 5400
gggtagcggt cgttgtccac tagggggtcc actcgctcca gggtgtgaag acacatgtcg 5460
ccctcttcgg catcaaggaa ggtgattggt ttgtaggtgt aggccacgtg accgggtgtt 5520
cctgaagggg ggctataaaa gggggtgggg gcgcgttcgt cctcactctc ttccgcatcg 5580
ctgtctgcga gggccagctg ttggggtgag tactccctct gaaaagcggg catgacttct 5640
gcgctaagat tgtcagtttc caaaaacgag gaggatttga tattcacctg gcccgcggtg 5700
atgcctttga gggtggccgc atccatctgg tcagaaaaga caatcttttt gttgtcaagc 5760
ttggtggcaa acgacccgta gagggcgttg gacagcaact tggcgatgga gcgcagggtt 5820
tggtttttgt cgcgatcggc gcgctccttg gccgcgatgt ttagctgcac gtattcgcgc 5880
gcaacgcacc gccattcggg aaagacggtg gtgcgctcgt cgggcaccag gtgcacgcgc 5940
caaccgcggt tgtgcagggt gacaaggtca acgctggtgg ctacctctcc gcgtaggcgc 6000
tcgttggtcc agcagaggcg gccgcccttg cgcgagcaga atggcggtag ggggtctagc 6060
tgcgtctcgt ccggggggtc tgcgtccacg gtaaagaccc cgggcagcag gcgcgcgtcg 6120
aagtagtcta tcttgcatcc ttgcaagtct agcgcctgct gccatgcgcg ggcggcaagc 6180
gcgcgctcgt atgggttgag tgggggaccc catggcatgg ggtgggtgag cgcggaggcg 6240
tacatgccgc aaatgtcgta aacgtagagg ggctctctga gtattccaag atatgtaggg 6300
tagcatcttc caccgcggat gctggcgcgc acgtaatcgt atagttcgtg cgagggagcg 6360
aggaggtcgg gaccgaggtt gctacgggcg ggctgctctg ctcggaagac tatctgcctg 6420
aagatggcat gtgagttgga tgatatggtt ggacgctgga agacgttgaa gctggcgtct 6480
gtgagaccta ccgcgtcacg cacgaaggag gcgtaggagt cgcgcagctt gttgaccagc 6540
tcggcggtga cctgcacgtc tagggcgcag tagtccaggg tttccttgat gatgtcatac 6600
ttatcctgtc cctttttttt ccacagctcg cggttgagga caaactcttc gcggtctttc 6660
cagtactctt ggatcggaaa cccgtcggcc tccgaacggt aagagcctag catgtagaac 6720
tggttgacgg cctggtaggc gcagcatccc ttttctacgg gtagcgcgta tgcctgcgcg 6780
gccttccgga gcgaggtgtg-ggtgagcgca aaggtgtccc tgaccatgac tttgaggtac 6840
tggtatttga agtcagtgtc gtcgcatccg ccctgctccc agagcaaaaa gtccgtgcgc 6900
tttttggaac gcggatttgg cagggcgaag gtgacatcgt tgaagagtat ctttcccgcg 6960
cgaggcataa agttgcgtgt gatgcggaag ggtcccggca cctcggaacg gttgttaatt 7020
acctgggcgg cgagcacgat ctcgtcaaag ccgttgatgt tgtggcccac aatgtaaagt 7080
tccaagaagc gcgggatgcc cttgatggaa ggcaattttt taagttcctc gtaggtgagc 7140
tcttcagggg agctgagccc gtgctctgaa agggcccagt ctgcaagatg agggttggaa 7200
gcgacgaatg agctccacag gtcacgggcc attagcattt gcaggtggtc gcgaaaggtc 7260
ctaaactggc gacctatggc cattttttct ggggtgatgc agtagaaggt aagcgggtct 7320
tgttcccagc ggtcccatcc aaggttcgcg gctaggtctc gcgcggcagt cactagaggc 7380
tcatctccgc cgaacttcat gaccagcatg aagggcacga gctgcttccc aaaggccccc 7440
atccaagtat aggtctctac atcgtaggtg acaaagagac gctcggtgcg aggatgcgag 7500
ccgatcggga agaactggat ctcccgccac caattggagg agtggctatt gatgtggtga 7560
aagtagaagt ccctgcgacg ggccgaacac tcgtgctggc ttttgtaaaa acgtgcgcag 7620
tactggcagc ggtgcacggg ctgtacatcc tgcacgaggt tgacctgacg accgcgcaca 7680
aggaagcaga gtgggaattt gagcccctcg cctggcgggt ttggctggtg gtcttctact 7740
tcggctgctt gtccttgacc gtctggctgc tcgaggggag ttacggtgga tcggaccacc 7800
acgccgcgcg agcccaaagt ccagatgtcc gcgcgcggcg gtcggagctt gatgacaaca 7860
tcgcgcagat gggagctgtc catggtctgg agctcccgcg gcgtcaggtc aggcgggagc 7920
tcctgcaggt ttacctcgca tagacgggtc agggcgcggg ctagatccag gtgataccta 7980
atttccaggg gctggttggt ggcggcgtcg atggcttgca agaggccgca tccccgcggc 8040
gcgactacgg taccgcgcgg cgggcggtgg gccgcggggg tgtccttgga tgatgcatct 8100
aaaagcggtg acgcgggcga gcccccggag gtaggggggg ctccggaccc gccgggagag 8160
ggggcagggg cacgtcggcg ccgcgcgcgg gcaggagctg gtgctgcgcg cgtaggttgc 8220
tggcgaacgc gacgacgcgg cggttgatct cctgaatctg gcgcctctgc gtgaagacga 8280

cgggcccggt gagcttgaac ctgaaagaga gttcgacaga atcaatttcg gtgtcgttga 8340
cggcggcctg gcgcaaaatc tcctgcacgt ctcctgagtt gtcttgatag gcgatctcgg 8400
ccatgaactg ctcgatctct tcctcctgga gatctccgcg tccggctcgc tccacggtgg 8460
cggcgaggtc gttggaaatg cgggccatga gctgcgagaa ggcgttgagg cctccctcgt 8520
tccagacgcg gctgtagacc acgccccctt cggcatcgcg ggcgcgcatg accacctgcg 8580
cgagattgag ctccacgtgc cgggcgaaga cggcgtagtt tcgcaggcgc tgaaagaggt 8640
agttgagggt ggtggcggtg tgttctgcca cgaagaagta cataacccag cgtcgcaacg 8700
tggattcgtt gatatccccc aaggcctcaa ggcgctccat ggcctcgtag aagtccacgg 8760
cgaagttgaa aaactgggag ttgcgcgccg acacggttaa ctcctcctcc agaagacgga 8820
tgagctcggc gacagtgtcg cgcacctcgc gctcaaaggc tacaggggcc tcttcttctt 8880
cttcaatctc ctcttccata agggcctccc cttcttcttc ttctggcggc ggtgggggag 8940
gggggacacg gcggcgacga cggcgcaccg ggaggcggtc gacaaagcgc tcgatcatct 9000
ccccgcggcg acggcgcatg gtctcggtga cggcgcggcc gttctcgcgg gggcgcagtt 9060
ggaagacgcc gcccgtcatg tcccggttat gggttggcgg ggggctgcca tgcggcaggg 9120
atacggcgct aacgatgcat ctcaacaatt gttgtgtagg tactccgccg ccgagggacc 9180
tgagcgagtc cgcatcgacc ggatcggaaa acctctcgag aaaggcgtct aaccagtcac 9240
agtcgcaagg taggctgagc accgtggcgg gcggcagcgg gcggcggtcg gggttgtttc 9300
tggcggaggt gctgctgatg atgtaattaa agtaggcggt cttgagacgg cggatggtcg 9360
acagaagcac catgtccttg ggtccggcct gctgaatgcg caggcggtcg gccatgcccc 9420
aggcttcgtt ttgacatcgg cgcaggtctt tgtagtagtc ttgcatgagc ctttctaccg 9480
gcacttcttc ttctccttcc tcttgtcctg catctcttgc atctatcgct gcggcggcgg 9540
cggagtttgg ccgtaggtgg cgccctcttc ctcccatgcg tgtgaccccg aagcccctca 9600
tcggctgaag cagggctagg tcggcgacaa cgcgctcggc taatatggcc tgctgcacct 9660
gcgtgagggt agactggaag tcatccatgt ccacaaagcg gtggtatgcg cccgtgttga 9720
tggtgtaagt gcagttggcc ataacggacc agttaacggt ctggtgaccc ggctgcgaga 9780
gctcggtgta cctgagacgc gagtaagccc tcgagtcaaa tacgtagtcg ttgcaagtcc 9840
gcaccaggta ctggtatccc accaaaaagt gcggcggcgg ctggcggtag aggggccagc 9900
gtagggtggc cggggctccg ggggcgagat cttccaacat aaggcgatga tatccgtaga 9960
tgtacctgga catccaggtg atgccggcgg cggtggtgga ggcgcgcgga aagtcgcgga 10020
cgcggttcca gatgttgcgc agcggcaaaa agtgctccat ggtcgggacg ctctggccgg 10080
tcaggcgcgc gcaatcgttg acgctctagc gtgcaaaagg agagcctgta agcgggcact 10140
cttccgtggt ctggtggata aattcgcaag ggtatcatgg cggacgaccg gggttcgagc 10200
cccgtatccg gccgtccgcc gtgatccatg cggttaccgc ccgcgtgtcg aacccaggtg 10260
tgcgacgtca gacaacgggg gagtgctcct tttggcttcc ttccaggcgc ggcggctgct 10320
gcgctagctt ttttggccac tggccgcgcg cagcgtaagc ggttaggctg gaaagcgaaa 10380
gcattaagtg gctcgctccc tgtagccgga gggttatttt ccaagggttg agtcgcggga 10440
cccccggttc gagtctcgga ccggccggac tgcggcgaac gggggtttgc ctccccgtca 10500
tgcaagaccc cgcttgcaaa ttcctccgga aacagggacg agcccctttt ttgcttttcc 10560
cagatgcatc cggtgctgcg gcagatgcgc ccccctcctc agcagcggca agagcaagag 10620
cagcggcaga catgcagggc accctcccct cctcctaccg cgtcaggagg ggcgacatcc 10680
gcggttgacg cggcagcaga tggtgattac gaacccccgc ggcgccgggc ccggcactac 10740
ctggacttgg aggagggcga gggcctggcg cggctaggag cgccctctcc tgagcggcac 10800
ccaagggtgc agctgaagcg tgatacgcgt gaggcgtacg tgccgcggca gaacctgttt 10860
cgcgaccgcg agggagagga gcccgaggag atgcgggatc gaaagttcca cgcagggcgc 10920
gagctgcggc atggcctgaa tcgcgagcgg ttgctgcgcg aggaggactt tgagcccgac 10980
gcgcgaaccg ggattagtcc cgcgcgcgca cacgtggcgg ccgccgacct ggtaaccgca 11040
tacgagcaga cggtgaacca ggagattaac tttcaaaaaa gctttaacaa ccacgtgcgt 11100
acgcttgtgg cgcgcgagga ggtggctata ggactgatgc atctgtggga ctttgtaagc 11160
gcgctggagc aaaacccaaa tagcaagccg ctcatggcgc agctgttcct tatagtgcag 11220
cacagcaggg acaacgaggc attcagggat gcgctgctaa acatagtaga gcccgagggc 11280

cgctggctgc tcgatttgat aaacatcctg cagagcatag tggtgcagga gcgcagcttg 11340
agcctggctg acaaggtggc cgccatcaac tattccatgc ttagcctggg caagttttac 11400
gcccgcaaga tataccatac cccttacgtt cccatagaca aggaggtaaa gatcgagggg 11460
ttctacatgc gcatggcgct gaaggtgctt accttgagcg acgacctggg cgtttatcgc 11520
aacgagcgca tccacaaggc cgtgagcgtg agccggcggc gcgagctcag cgaccgcgag 11580
ctgatgcaca gcctgcaaag ggccctggct ggcacgggca gcggcgatag agaggccgag 11640
tcctactttg acgcgggcgc tgacctgcgc tgggccccaa gccgacgcgc cctggaggca 11700
gctggggccg gacctgggct ggcggtggca cccgcgcgcg ctggcaacgt cggcggcgtg 11760
gaggaatatg acgaggacga tgagtacgag ccagaggacg gcgagtacta agcggtgatg 11820
tttctgatca gatgatgcaa gacgcaacgg acccggcggt gcgggcggcg ctgcagagcc 11880
agccgtccgg ccttaactcc acggacgact ggcgccaggt catggaccgc atcatgtcgc 11940
tgactgcgcg caatcctgac gcgttccggc agcagccgca ggccaaccgg ctctccgcaa 12000
ttctggaagc ggtggtcccg gcgcgcgcaa accccacgca cgagaaggtg ctggcgatcg 12060
taaacgcgct ggccgaaaac agggccatcc ggcccgacga ggccggcctg gtctacgacg 12120
cgctgcttca gcgcgtggct cgttacaaca gcggcaacgt gcagaccaac ctggaccggc 12180
tggtggggga tgtgcgcgag gccgtggcgc agcgtgagcg cgcgcagcag cagggcaacc 12240
tgggctccat ggttgcacta aacgccttcc tgagtacaca gcccgccaac gtgccgcggg 12300
gacaggagga ctacaccaac tttgtgagcg cactgcggct aatggtgact gagacaccgc 12360
aaagtgaggt gtaccagtct gggccagact attttttcca gaccagtaga caaggcctgc 12420
agaccgtaaa cctgagccag gctttcaaaa acttgcaggg gctgtggggg gtgcgggctc 12480
ccacaggcga ccgcgcgacc gtgtctagct tgctgacgcc caactcgcgc ctgttgctgc 12540
tgctaatagc gcccttcacg gacagtggca gcgtgtcccg ggacacatac ctaggtcact 12600
tgctgacact gtaccgcgag gccataggtc aggcgcatgt ggacgagcat actttccagg 12660
agattacaag tgtcagccgc gcgctggggc aggaggacac gggcagcctg gaggcaaccc 12720
taaactacct gctgaccaac cggcggcaga agatcccctc gttgcacagt ttaaacagcg 12780
aggaggagcg cattttgcgc tacgtgcagc agagcgtgag ccttaacctg atgcgcgacg 12840
gggtaacgcc cagcgtggcg ctggacatga ccgcgcgcaa catggaaccg ggcatgtatg 12900
cctcaaaccg gccgtttatc aaccgcctaa tggactactt gcatcgcgcg gccgccgtga 12960
accccgagta tttcaccaat gccatcttga acccgcactg gctaccgccc cctggtttct 13020
acaccggggg attcgaggtg cccgagggta acgatggatt cctctgggac gacatagacg 13080
acagcgtgtt ttccccgcaa ccgcagaccc tgctagagtt gcaacagcgc gagcaggcag 13140
aggcggcgct gcgaaaggaa agcttccgca ggccaagcag cttgtccgat ctaggcgctg 13200
cggccccgcg gtcagatgct agtagcccat ttccaagctt gatagggtct cttaccagca 13260
ctcgcaccac ccgcccgcgc ctgctgggcg aggaggagta cctaaacaac tcgctgctgc 13320
agccgcagcg cgaaaaaaac ctgcctccgg catttcccaa caacgggata gagagcctag 13380
tggacaagat gagtagatgg aagacgtacg cgcaggagca cagggacgtg ccaggcccgc 13440
gcccgcccac ccgtcgtcaa aggcacgacc gtcagcgggg tctggtgtgg gaggacgatg 13500
actcggcaga cgacagcagc gtcctggatt tgggagggag tggcaacccg tttgcgcacc 13560
ttcgccccag gctggggaga atgttttaaa aaaaaaaaaa gcatgatgca aaataaaaaa 13620
ctcaccaagg ccatggcacc gagcgttggt tttcttgtat tccccttagt atgcggcgcg 13680
cggcgatgta tgaggaaggt cctcctccct cctacgagag tgtggtgagc gcggcgccag 13740
tggcggcggc gctgggttct cccttcgatg ctcccctgga cccgccgttt gtgcctccgc 13800
ggtacctgcg gcctaccggg gggagaaaca gcatccgtta ctctgagttg gcacccctat 13860
tcgacaccac ccgtgtgtac ctggtggaca acaagtcaac ggatgtggca tccctgaact 13920
accagaacga ccacagcaac tttctgacca cggtcattca aaacaatgac tacagcccgg 13980
gggaggcaag cacacagacc atcaatcttg acgaccggtc gcactggggc ggcgacctga 14040
aaaccatcct gcataccaac atgccaaatg tgaacgagtt catgtttacc aataagttta 14100
aggcgcgggt gatggtgtcg cgcttgccta ctaaggacaa tcaggtggag ctgaaatacg 14160
agtgggtgga gttcacgctg cccgagggca actactccga gaccatgacc atagacctta 14220
tgaacaacgc gatcgtggag cactacttga aagtgggcag acagaacggg gttctggaaa 14280

gcgacatcgg ggtaaagttt gacacccgca acttcagact ggggtttgac cccgtcactg 14340
gtcttgtcat gcctggggta tatacaaacg aagccttcca tccagacatc attttgctgc 14400
caggatgcgg ggtggacttc acccacagcc gcctgagcaa cttgttgggc atccgcaagc 14460
ggcaaccctt ccaggagggc tttaggatca cctacgatga tctggagggt ggtaacattc 14520
ccgcactgtt ggatgtggac gcctaccagg cgagcttgaa agatgacacc gaacagggcg 14580
ggggtggcgc aggcggcagc aacagcagtg gcagcggcgc ggaagagaac tccaacgcgg 14640
cagccgcggc aatgcagccg gtggaggaca tgaacgatca tgccattcgc ggcgacacct 14700
ttgccacacg ggctgaggag aagcgcgctg aggccgaagc agcggccgaa gctgccgccc 14760
ccgctgcgca acccgaggtc gagaagcctc agaagaaacc ggtgatcaaa cccctgacag 14820
aggacagcaa gaaacgcagt tacaacctaa taagcaatga cagcaccttc acccagtacc 14880
gcagctggta ccttgcatac aactacggcg accctcagac cggaatccgc tcatggaccc 14940
tgctttgcac tcctgacgta acctgcggct cggagcaggt ctactggtcg ttgccagaca 15000
tgatgcaaga ccccgtgacc ttccgctcca cgcgccagat cagcaacttt ccggtggtgg 15060
gcgccgagct gttgcccgtg cactccaaga gcttctacaa cgaccaggcc gtctactccc 15120
aactcatccg ccagtttacc tctctgaccc acgtgttcaa tcgctttccc gagaaccaga 15180
ttttggcgcg cccgccagcc cccaccatca ccaccgtcag tgaaaacgtt cctgctctca 15240
cagatcacgg gacgctaccg ctgcgcaaca gcatcggagg agtccagcga gtgaccatta 15300
ctgacgccag acgccgcacc tgcccctacg tttacaaggc cctgggcata gtctcgccgc 15360
gcgtcctatc gagccgcact ttttgagcaa gcatgtccat ccttatatcg cccagcaata 15420
acacaggctg gggcctgcgc ttcccaagca agatgtttgg cggggccaag aagcgctccg 15480
accaacaccc agtgcgcgtg cgcgggcact accgcgcgcc ctggggcgcg cacaaacgcg 15540
gccgcactgg gcgcaccacc gtcgatgacg ccatcgacgc ggtggtggag gaggcgcgca 15600
actacacgcc cacgccgcca ccagtgtcca cagtggacgc ggccattcag accgtggtgc 15660
gcggagcccg gcgctatgct aaaatgaaga gacggcggag gcgcgtagca cgtcgccacc 15720
gccgccgacc cggcactgcc gcccaacgcg cggcggcggc cctgcttaac cgcgcacgtc 15780
gcaccggccg acgggcggcc atgcgggccg ctcgaaggct ggccgcgggt attgtcactg 15840
tgccccccag gtccaggcga cgagcggccg ccgcagcagc cgcggccatt agtgctatga 15900
ctcagggtcg caggggcaac gtgtattggg tgcgcgactc ggttagcggc ctgcgcgtgc 15960
ccgtgcgcac ccgccccccg cgcaactaga ttgcaagaaa aaactactta gactcgtact 16020
gttgtatgta tccagcggcg gcggcgcgca acgaagctat gtccaagcgc aaaatcaaag 16080
aagagatgct ccaggtcatc gcgccggaga tctatggccc cccgaagaag gaagagcagg 16140
attacaagcc ccgaaagcta aagcgggtca aaaagaaaaa gaaagatgat gatgatgaac 16200
ttgacgacga ggtggaactg ctgcacgcta ccgcgcccag gcgacgggta cagtggaaag 16260
gtcgacgcgt aaaacgtgtt ttgcgacccg gcaccaccgt agtctttacg cccggtgagc 16320
gctccacccg cacctacaag cgcgtgtatg atgaggtgta cggcgacgag gacctgcttg 16380
agcaggccaa cgagcgcctc ggggagtttg cctacggaaa gcggcataag gacatgctgg 16440
cgttgccgct ggacgagggc aacccaacac ctagcctaaa gcccgtaaca ctgcagcagg 16500
tgctgcccgc gcttgcaccg tccgaagaaa agcgcggcct aaagcgcgag tctggtgact 16560
tggcacccac cgtgcagctg atggtaccca agcgccagcg actggaagat gtcttggaaa 16620
aaatgaccgt ggaacctggg ctggagcccg aggtccgcgt gcggccaatc aagcaggtgg 16680
cgccgggact gggcgtgcag accgtggacg ttcagatacc cactaccagt agcaccagta 16740
ttgccaccgc cacagagggc atggagacac aaacgtcccc ggttgcctca gcggtggcgg 16800
atgccgcggt gcaggcggtc gctgcggccg cgtccaagac ctctacggag gtgcaaacgg 16860
acccgtggat gtttcgcgtt tcagcccccc ggcgcccgcg ccgttcgagg aagtacggcg 16920
ccgccagcgc gctactgccc gaatatgccc tacatccttc cattgcgcct acccccggct 16980
atcgtggcta cacctaccgc cccagaagac gagcaactac ccgacgccga accaccactg 17040
gaacccgccg ccgccgtcgc cgtcgccagc ccgtgctggc cccgatttcc gtgcgcaggg 17100
tggctcgcga aggaggcagg accctggtgc tgccaacagc gcgctaccac cccagcatcg 17160
tttaaaagcc ggtctttgtg gttcttgcag atatggccct cacctgccgc ctccgtttcc 17220
cggtgccggg attccgagga agaatgcacc gtaggagggg catggccggc cacggcctga 17280

cgggcggcat gcgtcgtgcg caccaccggc ggcggcgcgc gtcgcaccgt cgcatgcgcg 17340
gcggtatcct gcccctcctt attccactga tcgccgcggc gattggcgcc gtgcccggaa 17400
ttgcatccgt ggccttgcag gcgcagagac actgattaaa aacaagttgc atgtggaaaa 17460
atcaaaataa aaagtctgga ctctcacgct cgcttggtcc tgtaactatt ttgtagaatg 17520
gaagacatca actttgcgtc tctggccccg cgacacggct cgcgcccgtt catgggaaac 17580
tggcaagata tcggcaccag caatatgagc ggtggcgcct tcagctgggg ctcgctgtgg 17640
agcggcatta aaaatttcgg ttccaccgtt aagaactatg gcagcaaggc ctggaacagc 17700
agcacaggcc agatgctgag ggataagttg aaagagcaaa atttccaaca aaaggtggta 17760
gatggcctgg cctctggcat tagcggggtg gtggacctgg ccaaccaggc agtgcaaaat 17820
aagattaaca gtaagcttga tccccgccct cccgtagagg agcctccacc ggccgtggag 17880
acagtgtctc cagaggggcg tggcgaaaag cgtccgcgcc ccgacaggga agaaactctg 17940
gtgacgcaaa tagacgagcc tccctcgtac gaggaggcac taaagcaagg cctgcccacc 18000
acccgtccca tcgcgcccat ggctaccgga gtgctgggcc agcacacacc cgtaacgctg 18060
gacctgcctc cccccgccga cacccagcag aaacctgtgc tgccaggccc gaccgccgtt 18120
gttgtaaccc gtcctagccg cgcgtccctg cgccgcgccg ccagcggtcc gcgatcgttg 18180
cggcccgtag ccagtggcaa ctggcaaagc acactgaaca gcatcgtggg tctgggggtg 18240
caatccctga agcgccgacg atgcttctga tagctaacgt gtcgtatgtg tgtcatgtat 18300
gcgtccatgt cgccgccaga ggagctgctg agccgccgcg cgcccgcttt ccaagatggc 18360
taccccttcg atgatgccgc agtggtctta catgcacatc tcgggccagg acgcctcgga 18420
gtacctgagc cccgggctgg tgcagtttgc ccgcgccacc gagacgtact tcagcctgaa 18480
taacaagttt agaaccccca cggtggcgcc tacgcacgac gtgaccacag accggtccca 18540
gcgtttgacg ctgcggttca tccctgtgga ccgtgaggat actgcgtact cgtacaaggc 18600
gcggttcacc ctagctgtgg gtgataaccg tgtgctggac atggcttcca cgtactttga 18660
catccgcggc gtgctggaca ggggccctac ttttaagccc tactctggca ctgcctacaa 18720
cgccctggct cccaagggtg ccccaaatcc ttgcgaatgg gatgaagctg ctactgctct 18780
tgaaataaac ctagaagaag aggacgatga caacgaagac gaagtagacg agcaagctga 18840
gcagcaaaaa actcacgtat ttgggcaggc gccttattct ggtataaata ttacaaagga 18900
gggtattcaa ataggtgtcg aaggtcaaac acctaaatat gccgataaaa catttcaacc 18960
tgaacctcaa ataggagaat ctcagtggta cgaaacagaa attaatcatg cagctgggag 19020
agtcctaaaa aagactaccc caatgaaacc atgttacggt tcatatgcaa aacccacaaa 19080
tgaaaatgga gggcaaggca ttcttgtaaa gcaacaaaat ggaaagctag aaagtcaagt 19140
ggaaatgcaa tttttctcaa ctactgaggc agccgcaggc aatggtgata acttgactcc 19200
taaagtggta ttgtacagtg aagatgtaga tatagaaacc ccagacactc atatttctta 19260
catgcccact attaaggaag gtaactcacg agaactaatg ggccaacaat ctatgcccaa 19320
caggcctaat tacattgctt ttagggacaa ttttattggt ctaatgtatt acaacagcac 19380
gggtaatatg ggtgttctgg cgggccaagc atcgcagttg aatgctgttg tagatttgca 19440
agacagaaac acagagcttt cataccagct tttgcttgat tccattggtg atagaaccag 19500
gtacttttct atgtggaatc aggctgttga cagctatgat ccagatgtta gaattattga 19560
aaatcatgga actgaagatg aacttccaaa ttactgcttt ccactgggag gtgtgattaa 19620
tacagagact cttaccaagg taaaacctaa aacaggtcag gaaaatggat gggaaaaaga 19680
tgctacagaa ttttcagata aaaatgaaat aagagttgga aataattttg ccatggaaat 19740
caatctaaat gccaacctgt ggagaaattt cctgtactcc aacatagcgc tgtatttgcc 19800
cgacaagcta aagtacagtc cttccaacgt aaaaatttct gataacccaa acacctacga 19860
ctacatgaac aagcgagtgg tggctcccgg gctagtggac tgctacatta accttggagc 19920
acgctggtcc cttgactata tggacaacgt caacccattt aaccaccacc gcaatgctgg 19980
cctgcgctac cgctcaatgt tgctgggcaa tggtcgctat gtgcccttcc acatccaggt 20040
gcctcagaag ttctttgcca ttaaaaacct ccttctcctg ccgggctcat acacctacga 20100
gtggaacttc aggaaggatg ttaacatggt tctgcagagc tccctaggaa atgacctaag 20160
ggttgacgga gccagcatta agtttgatag catttgcctt tacgccacct tcttccccat 20220
ggcccacaac accgcctcca cgcttgaggc catgcttaga aacgacacca acgaccagtc 20280

ctttaacgac tatctctccg ccgccaacat gctctaccct atacccgcca acgctaccaa 20340
cgtgcccata tccatcccct cccgcaactg ggcggctttc cgcggctggg ccttcacgcg 20400
ccttaagact aaggaaaccc catcactggg ctcgggctac gacccttatt acacctactc 20460
tggctctata ccctacctag atggaacctt ttacctcaac cacaccttta agaaggtggc 20520
cattaccttt gactcttctg tcagctggcc tggcaatgac cgcctgctta cccccaacga 20580
gtttgaaatt aagcgctcag ttgacgggga gggttacaac gttgcccagt gtaacatgac 20640
caaagactgg ttcctggtac aaatgctagc taactataac attggctacc agggcttcta 20700
tatcccagag agctacaagg accgcatgta ctccttcttt agaaacttcc agcccatgag 20760
ccgtcaggtg gtggatgata ctaaatacaa ggactaccaa caggtgggca tcctacacca 20820
acacaacaac tctggatttg ttggctacct tgcccccacc atgcgcgaag gacaggccta 20880
ccctgctaac ttcccctatc cgcttatagg caagaccgca gttgacagca ttacccagaa 20940
aaagtttctt tgcgatcgca ccctttggcg catcccattc tccagtaact ttatgtccat 21000
gggcgcactc acagacctgg gccaaaacct tctctacgcc aactccgccc acgcgctaga 21060
catgactttt gaggtggatc ccatggacga gcccaccctt ctttatgttt tgtttgaagt 21120
ctttgacgtg gtccgtgtgc accagccgca ccgcggcgtc atcgaaaccg tgtacctgcg 21180
cacgcccttc tcggccggca acgccacaac ataaagaagc aagcaacatc aacaacagct 21240
gccgccatgg gctccagtga gcaggaactg aaagccattg tcaaagatct tggttgtggg 21300
ccatattttt tgggcaccta tgacaagcgc tttccaggct ttgtttctcc acacaagctc 21360
gcctgcgcca tagtcaatac ggccggtcgc gagactgggg gcgtacactg gatggccttt 21420
gcctggaacc cgcactcaaa aacatgctac ctctttgagc cctttggctt ttctgaccag 21480
cgactcaagc aggtttacca gtttgagtac gagtcactcc tgcgccgtag cgccattgct 21540
tcttcccccg accgctgtat aacgctggaa aagtccaccc aaagcgtaca ggggcccaac 21600
tcggccgcct gtggactatt ctgctgcatg tttctccacg cctttgccaa ctggccccaa 21660
actcccatgg atcacaaccc caccatgaac cttattaccg gggtacccaa ctccatgctc 21720
aacagtcccc aggtacagcc caccctgcgt cgcaaccagg aacagctcta cagcttcctg 21780
gagcgccact cgccctactt ccgcagccac agtgcgcaga ttaggagcgc cacttctttt 21840
tgtcacttga aaaacatgta aaaataatgt actagagaca ctttcaataa aggcaaatgc 21900
ttttatttgt acactctcgg gtgattattt acccccaccc ttgccgtctg cgccgtttaa 21960
aaatcaaagg ggttctgccg cgcatcgcta tgcgccactg gcagggacac gttgcgatac 22020
tggtgtttag tgctccactt aaactcaggc acaaccatcc gcggcagctc ggtgaagttt 22080
tcactccaca ggctgcgcac catcaccaac gcgtttagca ggtcgggcgc cgatatcttg 22140
aagtcgcagt tggggcctcc gccctgcgcg cgcgagttgc gatacacagg gttgcagcac 22200
tggaacacta tcagcgccgg gtggtgcacg ctggccagca cgctcttgtc ggagatcaga 22260
tccgcgtcca ggtcctccgc gttgctcagg gcgaacggag tcaactttgg tagctgcctt 22320
cccaaaaagg gcgcgtgccc aggctttgag ttgcactcgc accgtagtgg catcaaaagg 22380
tgaccgtgcc cggtctgggc gttaggatac agcgcctgca taaaagcctt gatctgctta 22440
aaagccacct gagcctttgc gccttcagag aagaacatgc cgcaagactt gccggaaaac 22500
tgattggccg gacaggccgc gtcgtgcacg cagcaccttg cgtcggtgtt ggagatctgc 22560
accacatttc ggccccaccg gttcttcacg atcttggcct tgctagactg ctccttcagc 22620
gcgcgctgcc cgttttcgct cgtcacatcc atttcaatca cgtgctcctt atttatcata 22680
atgcttccgt gtagacactt aagctcgcct tcgatctcag cgcagcggtg cagccacaac 22740
gcgcagcccg tgggctcgtg atgcttgtag gtcacctctg caaacgactg caggtacgcc 22800
tgcaggaatc gccccatcat cgtcacaaag gtcttgttgc tggtgaaggt cagctgcaac 22860
ccgcggtgct cctcgttcag ccaggtcttg catacggccg ccagagcttc cacttggtca 22920
ggcagtagtt tgaagttcgc ctttagatcg ttatccacgt ggtacttgtc catcagcgcg 22980
cgcgcagcct ccatgccctt ctcccacgca gacacgatcg gcacactcag cgggttcatc 23040
accgtaattt cactttccgc ttcgctgggc tcttcctctt cctcttgcgt ccgcatacca 23100
cgcgccactg ggtcgtcttc attcagccgc cgcactgtgc gcttacctcc tttgccatgc 23160
ttgattagca ccggtgggtt gctgaaaccc accatttgta gcgccacatc ttctctttct 23220
tcctcgctgt ccacgattac ctctggtgat ggcgggcgct cgggcttggg agaagggcgc 23280

ttctttttct tcttgggcgc aatggccaaa tccgccgccg aggtcgatgg ccgcgggctg 23340
ggtgtgcgcg gcaccagcgc gtcttgtgat gagtcttcct cgtcctcgga ctcgatacgc 23400
cgcctcatcc gcttttttgg gggcgcccgg ggaggcggcg gcgacgggga cggggacgac 23460
acgtcctcca tggttggggg acgtcgcgcc gcaccgcgtc cgcgctcggg ggtggtttcg 23520
cgctgctcct cttcccgact ggccatttcc ttctcctata ggcagaaaaa gatcatggag 23580
tcagtcgaga agaaggacag cctaaccgcc ccctctgagt tcgccaccac cgcctccacc 23640
gatgccgcca acgcgcctac caccttcccc gtcgaggcac ccccgcttga ggaggaggaa 23700
gtgattatcg agcaggaccc aggttttgta agcgaagacg acgaggaccg ctcagtacca 23760
acagaggata aaaagcaaga.ccaggacaac gcagaggcaa acgaggaaca agtcgggcgg 23820
ggggacgaaa ggcatggcga ctacctagat gtgggagacg acgtgctgtt gaagcatctg 23880
cagcgccagt gcgccattat ctgcgacgcg ttgcaagagc gcagcgatgt gcccctcgcc 23940
atagcggatg tcagccttgc ctacgaacgc cacctattct caccgcgcgt accccccaaa 24000
cgccaagaaa acggcacatg cgagcccaac ccgcgcctca acttctaccc cgtatttgcc 24060
gtgccagagg tgcttgccac ctatcacatc tttttccaaa actgcaagat acccctatcc 24120
tgccgtgcca accgcagccg agcggacaag cagctggcct tgcggcaggg cgctgtcata 24180
cctgatatcg cctcgctcaa cgaagtgcca aaaatctttg agggtcttgg acgcgacgag 24240
aagcgcgcgg caaacgctct gcaacaggaa aacagcgaaa atgaaagtca ctctggagtg 24300
ttggtggaac tcgagggtga caacgcgcgc ctagccgtac taaaacgcag catcgaggtc 24360
acccactttg cctacccggc acttaaccta ccccccaagg tcatgagcac agtcatgagt 24420
gagctgatcg tgcgccgtgc gcagcccctg gagagggatg caaatttgca agaacaaaca 24480
gaggagggcc tacccgcagt tggcgacgag cagctagcgc gctggcttca aacgcgcgag 24540
cctgccgact tggaggagcg acgcaaacta atgatggccg cagtgctcgt taccgtggag 24600
cttgagtgca tgcagcggtt ctttgctgac ccggagatgc agcgcaagct agaggaaaca 24660
ttgcactaca cctttcgaca gggctacgta cgccaggcct gcaagatctc caacgtggag 24720
ctctgcaacc tggtctccta ccttggaatt ttgcacgaaa accgccttgg gcaaaacgtg 24780
cttcattcca cgctcaaggg cgaggcgcgc cgcgactacg tccgcgactg cgtttactta 24840
tttctatgct acacctggca gacggccatg ggcgtttggc agcagtgctt ggaggagtgc 24900
aacctcaagg agctgcagaa actgctaaag caaaacttga aggacctatg gacggccttc 24960
aacgagcgct ccgtggccgc gcacctggcg gacatcattt tccccgaacg cctgcttaaa 25020
accctgcaac agggtctgcc agacttcacc agtcaaagca tgttgcagaa ctttaggaac 25080
tttatcctag agcgctcagg aatcttgccc gccacctgct gtgcacttcc tagcgacttt 25140
gtgcccatta agtaccgcga atgccctccg ccgctttggg gccactgcta ccttctgcag 25200
ctagccaact accttgccta ccactctgac ataatggaag acgtgagcgg tgacggtcta 25260
ctggagtgtc actgtcgctg caacctatgc accccgcacc gctccctggt ttgcaattcg 25320
cagctgctta acgaaagtca aattatcggt acctttgagc tgcagggtcc ctcgcctgac 25380
gaaaagtccg cggctccggg gttgaaactc actccggggc tgtggacgtc ggcttacctt 25440
cgcaaatttg tacctgagga ctaccacgcc cacgagatta ggttctacga agaccaatcc 25500
cgcccgccta atgcggagct taccgcctgc gtcattaccc agggccacat tcttggccaa 25560
ttgcaagcca tcaacaaagc ccgccaagag tttctgctac gaaagggacg gggggtttac 25620
ttggaccccc agtccggcga ggagctcaac ccaatccccc cgccgccgca gccctatcag 25680
cagcagccgc gggcccttgc ttcccaggat ggcacccaaa aagaagctgc agctgccgcc 25740
gccacccacg gacgaggagg aatactggga cagtcaggca gaggaggttt tggacgagga 25800
ggaggaggac atgatggaag actgggagag cctagacgag gaagcttccg aggtcgaaga 25860
ggtgtcagac gaaacaccgt caccctcggt cgcattcccc tcgccggcgc cccagaaatc 25920
ggcaaccggt tccagcatgg ctacaacctc cgctcctcag gcgccgccgg cactgcccgt 25980
tcgccgaccc aaccgtagat gggacaccac tggaaccagg gccggtaagt ccaagcagcc 26040
gccgccgtta gcccaagagc aacaacagcg ccaaggctac cgctcatggc gcgggcacaa 26100
gaacgccata gttgcttgct tgcaagactg tgggggcaac atctccttcg cccgccgctt 26160
tcttctctac catcacggcg tggccttccc ccgtaacatc ctgcattact accgtcatct 26220
ctacagccca tactgcaccg gcggcagcgg cagcaacagc agcggccaca cagaagcaaa 26280

ggcgaccgga tagcaagact ctgacaaagc ccaagaaatc cacagcggcg gcagcagcag 26340
gaggaggagc gctgcgtctg gcgcccaacg aacccgtatc gacccgcgag cttagaaaca 26400
ggatttttcc cactctgtat gctatatttc aacagagcag gggccaagaa caagagctga 26460
aaataaaaaa caggtctctg cgatccctca cccgcagctg cctgtatcac aaaagcgaag 26520
atcagcttcg gcgcacgctg gaagacgcgg aggctctctt cagtaaatac tgcgcgctga 26580
ctcttaagga ctagtttcgc gccctttctc aaatttaagc gcgaaaacta cgtcatctcc 26640
agcggccaca cccggcgcca gcacctgttg tcagcgccat tatgagcaag gaaattccca 26700
cgccctacat gtggagttac cagccacaaa tgggacttgc ggctggagct gcccaagact 26760
actcaacccg aataaactac atgagcgcgg gaccccacat gatatcccgg gtcaacggaa 26820
tacgcgccca ccgaaaccga attctcctgg aacaggcggc tattaccacc acacctcgta 26880
ataaccttaa tccccgtagt tggcccgctg ccctggtgta ccaggaaagt cccgctccca 26940
ccactgtggt acttcccaga gacgcccagg ccgaagttca gatgactaac tcaggggcgc 27000
agcttgcggg cggctttcgt cacagggtgc ggtcgcccgg gcagggtata actcacctga 27060
caatcagagg gcgaggtatt cagctcaacg acgagtcggt gagctcctcg cttggtctcc 27120
gtccggacgg gacatttcag atcggcggcg ccggccgctc ttcattcacg cctcgtcagg 27180
caatcctaac tctgcagacc tcgtcctctg agccgcgctc tggaggcatt ggaactctgc 27240
aatttattga ggagtttgtg ccatcggtct actttaaccc cttctcggga cctcccggcc 27300
actatccgga tcaatttatt cctaactttg acgcggtaaa ggactcggcg gacggctacg 27360
actgaatgtt ataagttcct gtccatccgc acccactatc ttcatgttgt tgcagatgaa 27420
gcgcgcaaga ccgtctgaag ataccttcaa ccccgtgtat ccatatgaca cggaaaccgg 27480
tcctccaact gtgccttttc ttactcctcc ctttgtatcc cccaatgggt ttcaagagag 27540
tccccctggg gtactctctt tgcgcctatc cgaacctcta gttacctcca atggcatgct 27600
tgcgctcaaa atgggcaacg gcctctctct ggacgaggcc ggcaacctta cctcccaaaa 27660
tgtaaccact gtgagcccac ctctcaaaaa aaccaagtca aacataaacc tggaaatatc 27720
tgcacccctc acagttacct cagaagccct aactgtggct gccgccgcac ctctaatggt 27780
cgcgggcaac acactcacca tgcaatcaca ggccccgcta accgtgcacg actccaaact 27840
tagcattgcc acccaaggac ccctcacagt gtcagaagga aagctagccc tgcaaacatc 27900
aggccccctc accaccaccg atagcagtac ccttactatc actgcctcac cccctctaac 27960
tactgccact ggtagcttgg gcattgactt gaaagagccc atttatacac aaaatggaaa 28020
actaggacta aagtacgggg ctcctttgca tgtaacagac gacctaaaca ctttgaccgt 28080
agcaactggt ccaggtgtga ctattaataa tacttccttg caaactaaag ttactggagc 28140
cttgggtttt gattcacaag gcaatatgca acttaatgta gcaggaggac taaggattga 28200
ttctcaaaac agacgcctta tacttgatgt tagttatccg tttgatgctc aaaaccaact 28260
aaatctaaga ctaggacagg gccctctttt tataaactca gcccacaact tggatattaa 28320
ctacaacaaa ggcctttact tgtttacagc ttcaaacaat tccaaaaagc ttgaggttaa 28380
cctaagcact gccaaggggt tgatgtttga cgctacagcc atagccatta atgcaggaga 28440
tgggcttgaa tttggttcac ctaatgcacc aaacacaaat cccctcaaaa caaaaattgg 28500
ccatggccta gaatttgatt caaacaaggc tatggttcct aaactaggaa ctggccttag 28560
ttttgacagc acaggtgcca ttacagtagg aaacaaaaat aatgataagc taactttgtg 28620
gaccacacca gctccatctc ctaactgtag actaaatgca gagaaagatg ctaaactcac 28680
tttggtctta acaaaatgtg gcagtcaaat acttgctaca gtttcagttt tggctgttaa 28740
aggcagtttg gctccaatat ctggaacagt tcaaagtgct catcttatta taagatttga 28800
cgaaaatgga gtgctactaa acaattcctt cctggaccca gaatattgga actttagaaa 28860
tggagatctt actgaaggca cagcctatac aaacgctgtt ggatttatgc ctaacctatc 28920
agcttatcca aaatctcacg gtaaaactgc caaaagtaac attgtcagtc aagtttactt 28980
aaacggagac aaaactaaac ctgtaacact aaccattaca ctaaacggta cacaggaaac 29040
aggagacaca actccaagtg catactctat gtcattttca tgggactggt ctggccacaa 29100
ctacattaat gaaatatttg ccacatcctc ttacactttt tcatacattg cccaagaata 29160
aagaatcgtt tgtgttatgt ttcaacgtgt ttatttttca attgcagaaa atttcaagtc 29220
atttttcatt cagtagtata gccccaccac cacatagctt atacagatca ccgtacctta 29280

atcaaactca cagaacccta gtattcaacc tgccacctcc ctcccaacac acagagtaca 29340
cagtcctttc tccccggctg gccttaaaaa gcatcatatc atgggtaaca gacatattct 29400
taggtgttat attccacacg gtttcctgtc gagccaaacg ctcatcagtg atattaataa 29460
actccccggg cagctcactt aagttcatgt cgctgtccag ctgctgagcc acaggctgct 29520
gtccaacttg cggttgctta acgggcggcg aaggagaagt ccacgcctac atgggggtag 29580
agtcataatc gtgcatcagg atagggcggt ggtgctgcag cagcgcgcga ataaactgct 29640
gccgccgccg ctccgtcctg caggaataca acatggcagt ggtctcctca gcgatgattc 29700
gcaccgcccg cagcataagg cgccttgtcc tccgggcaca gcagcgcacc ctgatctcac 29760
ttaaatcagc acagtaactg cagcacagca ccacaatatt gttcaaaatc ccacagtgca 29820
aggcgctgta tccaaagctc atggcgggga ccacagaacc cacgtggcca tcataccaca 29880
agcgcaggta gattaagtgg cgacccctca taaacacgct ggacataaac attacctctt 29940
ttggcatgtt gtaattcacc acctcccggt accatataaa cctctgatta aacatggcgc 30000
catccaccac catcctaaac cagctggcca aaacctgccc gccggctata cactgcaggg 30060
aaccgggact ggaacaatga cagtggagag cccaggactc gtaaccatgg atcatcatgc 30120
tcgtcatgat atcaatgttg gcacaacaca ggcacacgtg catacacttc ctcaggatta 30180
caagctcctc ccgcgttaga accatatccc agggaacaac ccattcctga atcagcgtaa 30240
atcccacact gcagggaaga cctcgcacgt aactcacgtt gtgcattgtc aaagtgttac 30300
attcgggcag cagcggatga tcctccagta tggtagcgcg ggtttctgtc tcaaaaggag 30360
gtagacgatc cctactgtac ggagtgcgcc gagacaaccg agatcgtgtt ggtcgtagtg 30420
tcatgccaaa tggaacgccg gacgtagtca tatttcctga agcaaaacca ggtgcgggcg 30480
tgacaaacag atctgcgtct ccggtctcgc cgcttagatc gctctgtgta gtagttgtag 30540
tatatccact ctctcaaagc atccaggcgc cccctggctt cgggttctat gtaaactcct 30600
tcatgcgccg ctgccctgat aacatccacc accgcagaat aagccacacc cagccaacct 30660
acacattcgt tctgcgagtc acacacggga ggagcgggaa gagctggaag aaccatgttt 30720
ttttttttat tccaaaagat tatccaaaac ctcaaaatga agatctatta agtgaacgcg 30780
ctcccctccg gtggcgtggt caaactctac agccaaagaa cagataatgg catttgtaag 30840
atgttgcaca atggcttcca aaaggcaaac ggccctcacg tccaagtgga cgtaaaggct 30900
aaacccttca gggtgaatct cctctataaa cattccagca ccttcaacca tgcccaaata 30960
attctcatct cgccaccttc tcaatatatc tctaagcaaa tcccgaatat taagtccggc 31020
cattgtaaaa atctgctcca gagcgccctc caccttcagc ctcaagcagc gaatcatgat 31080
tgcaaaaatt caggttcctc acagacctgt ataagattca aaagcggaac attaacaaaa 31140
ataccgcgat cccgtaggtc ccttcgcagg gccagctgaa cataatcgtg caggtctgca 31200
cggaccagcg cggccacttc cccgccagga accatgacaa aagaacccac actgattatg 31260
acacgcatac tcggagctat gctaaccagc gtagccccga tgtaagcttg ttgcatgggc 31320
ggcgatataa aatgcaaggt gctgctcaaa aaatcaggca aagcctcgcg caaaaaagaa 31380
agcacatcgt agtcatgctc atgcagataa aggcaggtaa gctccggaac caccacagaa 31440
aaagacacca tttttctctc aaacatgtct gcgggtttct gcataaacac aaaataaaat 31500
aacaaaaaaa catttaaaca ttagaagcct gtcttacaac aggaaaaaca acccttataa 31560
gcataagacg gactacggcc atgccggcgt gaccgtaaaa aaactggtca ccgtgattaa 31620
aaagcaccac cgacagctcc tcggtcatgt ccggagtcat aatgtaagac tcggtaaaca 31680
catcaggttg attcacatcg gtcagtgcta aaaagcgacc gaaatagccc gggggaatac 31740
atacccgcag gcgtagagac aacattacag cccccatagg aggtataaca aaattaatag 31800
gagagaaaaa cacataaaca cctgaaaaac cctcctgcct aggcaaaata gcaccctccc 31860
gctccagaac aacatacagc gcttccacag cggcagccat aacagtcagc cttaccagta 31920
aaaaagaaaa cctattaaaa aaacaccact cgacacggca ccagctcaat cagtcacagt 31980
gtaaaaaagg gccaagtgca gagcgagtat atataggact aaaaaatgac gtaacggtta 32040
aagtccacaa aaaacaccca gaaaaccgca cgcgaaccta cgcccagaaa cgaaagccaa 32100
aaaacccaca acttcctcaa atcgtcactt ccgttttccc acgttacgtc acttcccatt 32160
ttaagaaaac tacaattccc aacacataca agttactccg ccctaaaacc tacgtcaccc 32220
gccccgttcc cacgccccgc gccacgtcac aaactccacc ccctcattat catattggct 32280

tcaatccaaa ataaggtata ttattgatga tgttacatcg ttaattaacg atttcgaacc 32340
cggggtaccg aattcctcga gtctagagga gcatgcgacg tcgcaattcg ccctatagtg 32400
agtcgtatta caattcactg gccgtcgttt tacaacgtcg tgactgggaa aaccctggcg 32460
ttacccaact taatcgcctt gcagcacatc cccctttcgc cagctggcgt aatagcgaag 32520
aggcccgcac cgatcgccct tcccaacagt tgcgcagcct gaatggcgaa tggaaattgt 32580
aagcgttaat attttgttaa aattcgcgtt aaatttttgt taaatcagct cattttttaa 32640
ccaataggcc gaaatcggca aaatccctta taaatcaaaa gaatagaccg agatagggtt 32700
gagtgttgtt ccagtttgga acaagagtcc actattaaag aacgtggact ccaacgtcaa 32760
agggcgaaaa accgtctatc agggcgatgg cccactacgt gaaccatcac cctaatcaag 32820
ttttttgggg tcgaggtgcc gtaaagcact aaatcggaac cctaaaggga gcccccgatt 32880
tagagcttga cggggaaagc cggcgaacgt ggcgagaaag gaagggaaga aagcgaaagg 32940
agcgggcgct agggcgctgg caagtgtagc ggtcacgctg cgcgtaacca ccacacccgc 33000
cgcgcttaat gcgccgctac agggcgcgtc ctgatgcggt attttctcct tacgcatctg 33060
tgcggtattt cacaccgcat acaggtggca cttttcgggg aaatgtgcgc ggaaccccta 33120
tttgtttatt tttctaaata cattcaaata tgtatccgct catgagacaa taaccctgat 33180
aaatgcttca ataatattga aaaaggaaga gtatgagtat tcaacatttc cgtgtcgccc 33240
ttattccctt ttttgcggca ttttgccttc ctgtttttgc tcacccagaa acgctggtga 33300
aagtaaaaga tgctgaagat cagttgggtg cacgagtggg ttacatcgaa ctggatctca 33360
acagcggtaa gatccttgag agttttcgcc ccgaagaacg ttttccaatg atgagcactt 33420
ttaaagttct gctatgtggc gcggtattat cccgtattga cgccgggcaa gagcaactcg 33480
gtcgccgcat acactattct cagaatgact tggttgagta ctcaccagtc acagaaaagc 33540
atcttacgga tggcatgaca gtaagagaat tatgcagtgc tgccataacc atgagtgata 33600
acactgcggc caacttactt ctgacaacga tcggaggacc gaaggagcta accgcttttt 33660
tgcacaacat gggggatcat gtaactcgcc ttgatcgttg ggaaccggag ctgaatgaag 33720
ccataccaaa cgacgagcgt gacaccacga tgcctgtagc aatggcaaca acgttgcgca 33780
aactattaac tggcgaacta cttactctag cttcccggca acaattaata gactggatgg 33840
aggcggataa agttgcagga ccacttctgc gctcggccct tccggctggc tggtttattg 33900
ctgataaatc tggagccggt gagcgtgggt ctcgcggtat cattgcagca ctggggccag 33960
atggtaagcc ctcccgtatc gtagttatct acacgacggg gagtcaggca actatggatg 34020
aacgaaatag acagatcgct gagataggtg cctcactgat taagcattgg taactgtcag 34080
accaagttta ctcatatata ctttagattg atttaaaact tcatttttaa tttaaaagga 34140
tctaggtgaa gatccttttt gataatctca tgaccaaaat cccttaacgt gagttttcgt 34200
tccactgagc gtcagacccc gtagaaaaga tcaaaggatc ttcttgagat cctttttttc 34260
tgcgcgtaat ctgctgcttg caaacaaaaa aaccaccgct accagcggtg gtttgtttgc 34320
cggatcaaga gctaccaact ctttttccga aggtaactgg cttcagcaga gcgcagatac 34380
caaatactgt tcttctagtg tagccgtagt taggccacca cttcaagaac tctgtagcac 34440
cgcctacata cctcgctctg ctaatcctgt taccagtggc tgctgccagt ggcgataagt 34500
cgtgtcttac cgggttggac tcaagacgat agttaccgga taaggcgcag cggtcgggct 34560
gaacgggggg ttcgtgcaca cagcccagct tggagcgaac gacctacacc gaactgagat 34620
acctacagcg tgagctatga gaaagcgcca cgcttcccga agggagaaag gcggacaggt 34680
atccggtaag cggcagggtc.ggaacaggag agcgcacgag ggagcttcca gggggaaacg 34740
cctggtatct ttatagtcct gtcgggtttc gccacctctg acttgagcgt cgatttttgt 34800
gatgctcgtc aggggggcgg agcctatgga aaaacgccag caacgcggcc tttttacggt 34860
tcctggcctt ttgctggcct tttgctcaca tgttctttcc tgcgttatcc cctgattctg 34920
tggataaccg tattaccgcc tttgagtgag ctgataccgc tcgccgcagc cgaacgaccg 34980
agcgcagcga gtcagtgagc gaggaagcgg aagagcgccc aatacgcaaa ccgcctctcc 35040
ccgcgcgttg gccgattcat taatgcagct ggcacgacag gtttcccgac tggaaagcgg 35100
gcagtgagcg caacgcaatt aatgtgagtt agctcactca ttaggcaccc caggctttac 35160
actttatgct tccggctcgt atgttgtgtg gaattgtgag cggataacaa tttcacacag 35220
gaaacagcta tgaccatgat tacgccaagc tatttaggtg acactataga atactcaagc 35280

tagttaatta acgttaatta acatcatcaa taatatacct tattttggat tgaagccaat 35340
atgataatga gggggtggag tttgtgacgt ggcgcggggc gtgggaacgg ggcgggtgac 35400
gtagtagtgt ggcggaagtg tgatgttgca agtgtggcgg aacacatgta agcgacggat 35460
gtggcaaaag tgacgttttt ggtgtgcgcc ggtgtacaca ggaagtgaca attttcgcgc 35520
ggttttaggc ggatgttgta gtaaatttgg gcgtaaccga gtaagatttg gccattttcg 35580
cgggaaaact gaataagagg aagtgaaatc tgaataattt tgtgttactc atagcgcgta 35640
atctctagca t 35651
<212> Type : DNA
<211> Length : 35651
SequenceName : SEQ ID No.1
SequenceDescription :

Custom Codon
------------
Sequence Name : SEQ ID No.1

### REFERENCES

1. Andersen, M. M. and T. Ronne. 1991. Side-effects with Japanese encephalitis vaccine. Lancet 337:1044.
2. Ashok, M. S. and P. N. Rangarajan. 2000. Immunization with plasmid DNA encoding the envelope glycoprotein of Japanese encephalitis virus confers significant protection against intracerebral viral challenge without inducing detectable antiviral antibodies. Vaccine 18:68-75.
3. Both, G. W., L. J. Lockett, V. Janardhana, S. J. Edwards, A. R. Bellamy, F. L. Graham, L. Prevec, and M. E. Andrew. 1993. Protective immunity to rotavirus-induced diarrhoea is passively transferred to newborn mice from naive dams vaccinated with a single dose of a recombinant adenovirus expressing rotavirus VP7sc. Virology 193:940-950.
4. Casimiro, D. R., A. Tang, L. Chen, T. M. Fu, R. K. Evans, M. E. Davies, D. C. Freed, W. Hurni, J. M. Aste-Amezaga, L. Guan, R. Long, L. Huang, V. Harris, D. K. Nawrocki, H. Mach, R. D. Troutman, L. A. Isopi, K. K. Murthy, K. Rice, K. A. Wilson, D. B. Volkin, E. A. Emini, and J. W. Shiver. 2003. Vaccine-induced immunity in baboons by using DNA and replication-incompetent adenovirus type 5 vectors expressing a human immunodeficiency virus type 1 gag gene. J Virol. 77:7663-7668.
5. Chambers, T. J., C. S. Hahn, R. Galler, and C. M. Rice. 1990. Flavivirus genome organization, expression and replication. Annu. Rev. Microbiol. 44:649.
6. Chang, G.-J. J., A. R. Hunt, and B. Davis. 2000. A single intramuscular injection of recombinant plasmid DNA induces protective immunity and prevents Japanese encephalitis in mice. Journal of Virology 74:4244-4252.
7. Chen, H. W., C. H. Pan, M. Y. Liau, R. Jou, C. J. Tsai, H. J. Wu, Y. L. Lin, and M. H. Tao. 1999. Screening of protective antigens of Japanese encephalitis virus by DNA immunization: a comparative study with conventional viral vaccines. J. Virol. 73:10137-10145.
8. Chengalvala, M. V., B. M. Bhat, R. A. Bhat, S. K. Deer, M. D. Lubeck, R. H. Purcell, and K. K. Murthy. 1997. Replication and immunogenicity of Ad7-, Ad4-, and Ad5-hepatitis B virus surface antigen recombinants, with or without a portion of E3 region, in chimpanzees. Vaccine 15:335-339.
9. Flanagan, B., C. R. Pringle, and K. N. Leppard. 1997. A recombinant human adenovirus expressing the simian immunodeficiency virus Gag antigen can induce long-lived immune responses in mice. J Gen. Virol. 78 ( Pt 5):991-997.
10. Fonseca, B. A., S. Pincus, R. E. Shope, E. Paoletti, and P. W. Mason. 1994. Recombinant vaccinia viruses co-expressing dengue-1 glycoproteins prM and E induce neutralizing antibodies in mice. Vaccine 12:279-285.
11. Fooks, A. R., D. Jeevarajah, J. Lee, A. Warnes, S. Niewiesk, M. ter, V, J. R. Stephenson, and J. C. Clegg. 1998. Oral or parenteral administration of replication-deficient adenoviruses expressing the measles virus haemagglutinin and fusion proteins: protective immune responses in rodents. J Gen. Virol. 79 ( Pt 5):1027-1031.
12. Guirakhoo, F., R. Weltzin, T. J. Chambers, Z. X. Zhang, K. Soike, M. Ratterree, J. Arroyo, K. Georgakopoulos, J. Catalan, and T. P. Monath. 2000. Recombinant chimeric yellow fever-dengue type 2 virus is immunogenic and protective in nonhuman primates. J Virol. 74:5477-5485.
13. Imler J.L. 1995. Adenovirus vectors as recombinant viral vccines. Vaccine 13:1143-1151.
14. Jan, L. R., C. S. Yang, L. S. Henchal, H. Sumiyoshi, P. L. Summers, D. R. Dubois, and C. J. Lai. 1993. Increased immunogenicity and protective efficacy in outbred and inbred mice by strategic carboxyl-terminal truncation of Japanese encephalitis virus envelope glycoprotein. Am. J Trop. Med. Hyg. 48:412-423.
15. Kaur, R., G. Sachdeva, and S. Vrati. 2002. Plasmid DNA immunization against Japanese Encephalitis Virus: immunogenicity of membrane-anchored and secretory envelope protein. J. Infect. Dis. 185:1-12.
16. Kaur, R. and S. Vrati. 2003. Development of a recombinant vaccine against Japanese encephalitis. J Neurovirol 9:421-431.
17. Kimura-Kiroda, J. and K. Yasui. 1988. Protection of mice against Japanese encephalitis virus by passive administration of monoclonal antibodies. J. Immunol.141:3 606-3610.
18. Konishi, E., S. Pincus, E. Paoletti, R. E. Shope, T. Burrage, and P. W. Mason. 1992. Mice immunized with a subviral particle containing the Japanese encephalitis virus prM/M and E proteins are protected from lethal JEV infection. Virology 188:714-720.
19. Konishi, E., M. Yamaoka, S. W. Khin, I. Kurane, K. Takada, and P. W. Mason. 1999. The anamnestic neutralizing antibody response is critical for protection of mice from challenge following vaccination with a plasmid encoding the Japanese encephalitis virus premembrane and envelope genes. J. Virol. 73:5527-5534.
20. Konishi, E., M. Yamaoka, Khin-Sane-Win, I. Kurane, and Mason P.W. 1998. Induction of protective immunity against Japanese encephaltis in mice by immunization with a plasmid encoding Japanese encephalitis virus premembrane and envelope genes. Journal of Virology 72:4925-4930.
21. Lemiale, F., W. P. Kong, L. M. Akyurek, X. Ling, Y. Huang, B. K. Chakrabarti, M. Eckhaus, and G. J. Nabel. 2003. Enhanced mucosal immunoglobulin A response of intranasal adenoviral vector human immunodeficiency virus vaccine and localization in the central nervous system. J Virol. 77:10078-10087.
22. Lin, B., C. R. Parrish, J. M. Murray, and P. J. Wright. 1994. Localization of a neutralizing epitope on the envelope protein of dengue virus type 2. Virology 202:885-890.
23. Livingston, P. G., I. Kurane, C. J. Lai, M. Bray, and F. A. Ennis. 1994. Recognition of envelope protein by dengue virus serotype-specific human CD4+ CD8- cytotoxic T-cell clones: J Virol. 68:3283-3288.
24. Lubeck, M. D., A. R. Davis, M. Chengalvala, R. J. Natuk, J. E. Morin, K. Molnar-Kimber, B. B. Mason, B. M. Bhat, S. Mizutani, P. P. Hung, and. 1989. Immunogenicity and efficacy testing in chimpanzees of an oral hepatitis B vaccine based on live recombinant adenovirus. Proc. Natl. Acad. Sci. U. S. A 86:6763-6767.
25. Mason P.W., S. Pincus, M. J. Fournier, T. L. Mason, R. E. Shope, and E. Paoletti. 1991. Japanese encephalitis virus-Vaccinia recombinants produce particulate forms of the structural membrane proteins and induce high levels of protection against lethal JEV infection. Virology 180:294-305.
26. McMinn, P. C. 1997. The molecular basis of virulence of the encephalitogenic flaviviruses. J Gen. Virol. 78 ( Pt 11):2711-2722.
27. Men, R., M. Bray, and C.-J. Lai. 1991. Carboxy-terminal truncated dengue virus envelope glycoprotein expressed on the cell surface and secreted intracellularly exhibit increased immunogenicity in mice. Journal of Virology 65:1400-1407.
28. Monath, T. P. 2001. Prospects for development of a vaccine against the West Nile virus. Ann. N. Y. Acad. Sci. 951:1-12.
29. Monath, T. P. 2002. Japanese encephalitis vaccines: current vaccines and future prospects. Curr. Top. Microbiol. Immunol. 267:105-138.
30. Monath, T. P., K. McCarthy, P. Bedford, C. T. Johnson, R. Nichols, S. Yoksan, R. Marchesani, M. Knauber, K. H. Wells, J. Arroyo, and F. Guirakhoo. 2002. Clinical proof of principle for ChimeriVax: recombinant live, attenuated vaccines against flavivirus infections. Vaccine 20:1004-1018.
31. Monath, T. P., K. Soike, I. Levenbook, Z. X. Zhang, J. Arroyo, S. Delagrave, G. Myers, A. D. Barrett, R. E. Shope, M. Ratterree, T. J. Chambers, and F. Guirakhoo. 1999. Recombinant, chimaeric live, attenuated vaccine (ChimeriVax) incorporating the envelope genes of Japanese encephalitis (SA14-14-2) virus and the capsid and nonstructural genes of yellow fever (17D) virus is safe, immunogenic and protective in non-human primates. Vaccine 17:1869-1882.
32. Pan, C. H., H. W. Chen, H. W. Huang, and M. H. Tao. 2001. Protective mechanisms induced by a Japanese encephalitis virus DNA vaccine: requirement for antibody but not CD8(+) cytotoxic T-cell responses. J Virol. 75:11457-11463.
33. Plesner, A. M. and T. Ronne. 1997. Allergic mucocutaneous reactions to Japanese encephalitis vaccine. Vaccine 15:1239-1243.
34. Putnak, R., R. Feighny, J. Burrous, M. Cochran, C. Hackett, G. Smith, and C. Hoke. 1991. Dengue-1 virus envelope glycoprotein gene expressed in recombinant baculovirus elicits virus-neutralizing antibody in mice and protects them from virus challenge. Am. J Trop. Med. Hyg. 45:159-167.
35. Raikwar, S. P., P. Malik, O. Singh, and S. Vrati. 1997. Recombinant adenovirus synthesizing cell surface-anchored beta hCG induces bioneutralizing antibodies in rats. Gene 190:197-202.
36. Ramakrishna, C., A. Desai, S. K. Shankar, A. Chandramuki, and V. Ravi. 1999. Oral immunisation of mice with live Japanese encephalitis virus induces a protective immune response. Vaccine 17:3102-3108.
37. Raviprakash, K., T. J. Kochel, D. Ewing, M. Simmons, I. Phillips, C. G. Hayes, and K. R. Porter. 2000. Immunogenicity of dengue virus type 1 DNA vaccines expressing truncated and full length envelope protein. Vaccine 18:2426-2434.
38. Ruff, T. A., D. Eisen, A. Fuller, and R. Kas. 1991. Adverse reactions to Japanese encephalitis vaccine. Lancet 338:881-882.
39. Sakaguchi, M., M. Yoshida, W. Kuroda, O. Harayama, Y. Matsunaga, and S. Inouye. 2000. Systemic immediate-type reactions to gelatin included in Japanese encephalitis vaccines. Vaccine 15:121-122.
40. Sharpe, S., A. Fooks, J. Lee, K. Hayes, C. Clegg, and M. Cranage. 2002. Single oral immunization with replication deficient recombinant adenovirus elicits long-lived transgene-specific cellular and humoral immune responses. Virology 293:210-216.
41. Shiver, J. W., T. M. Fu, L. Chen, D. R. Casimiro, M. E. Davies, R. K. Evans, Z. Q. Zhang, A. J. Simon, W. L. Trigona, S. A. Dubey, L. Huang, V. A. Harris, R. S. Long, X. Liang, L. Handt, W. A. Schleif, L. Zhu, D. C. Freed, N. V. Persaud, L. Guan, K. S. Punt, A. Tang, M. Chen, K. A. Wilson, K. B. Collins, G. J. Heidecker, V. R. Fernandez, H. C. Perry, J. G. Joyce, K. M. Grimm, J. C. Cook, P. M. Keller, D. S. Kresock, H. Mach, R. D. Troutman, L. A. Isopi, D. M. Williams, Z. Xu, K. E. Bohannon, D. B. Volkin, D. C. Montefiori, A. Miura, G. R. Krivulka, M. A. Lifton, M. J. Kuroda, J. E. Schmitz, N. L. Letvin, M. J. Caulfield, A. J. Bett, R. Youil, D. C. Kaslow, and E. A. Emini. 2002. Replication-incompetent adenoviral vaccine vector elicits effective anti-immunodeficiency-virus immunity. Nature 415:331-335.
42. Vos, A., A. Neubert, E. Pommerening, T. Muller, L. Dohner, L. Neubert, and K. Hughes. 2001. Immunogenicity of an E1-deleted recombinant human adenovirus against rabies by different routes of administration. J Gen. Virol. 82:2191-2197.
43. Vrati, S., V. Agarwal, P. Malik, S. A. Wani, and M. Saini. 1999. Molecular characterization of an Indian isolate of Japanese encephalitis virus that shows an extended lag phase during growth. J. Gen. Virol. 80:1665-1671.
44. Vrati, S., R. K. Giri, A. Razdan, and P. Malik. 1999. Complete nucleotide sequence of an Indian strain of Japanese encephalitis virus: sequence comparison with other strains and phylogenetic analysis. Am. J. Trop. Med. Hyg. 61:677-680.
45. World Health Organization. 1998. Japanese encephalitis vaccines. Wkly Epidemiol Rec 73:334-344.
46. Xiang, Z., Y. Li, G. Gao, J. M. Wilson, and H. C. Ertl. 2003. Mucosally delivered E1-deleted adenoviral vaccine carriers induce transgene product-specific antibody responses in neonatal mice. J Immunol. 171:4287-4293.
47. Xiang, Z. Q., G. P. Gao, A. Reyes-Sandoval, Y. Li, J. M. Wilson, and H. C. Ertl. 2003. Oral vaccination of mice with adenoviral vectors is not impaired by preexisting immunity to the vaccine carrier. J Virol. 77:10780-10789.
48. Xiang, Z. Q., Y. Yang, J. M. Wilson, and H. C. Ertl. 1996. A replication-defective human adenovirus recombinant serves as a highly efficacious vaccine carrier. Virology 219:220-227.
49. Yoshida, T., K. Okuda, K. Q. Xin, K. Tadokoro, J. Fukushima, S. Toda, E. Hagiwara, K. Hamajima, T. Koshino, and T. Saito. 2001. Activation of HIV-1-specific immune responses to an HIV-1 vaccine constructed from a replication-defective adenovirus vector using various combinations of immunization protocols. Clin. Exp. Immunol. 124:445-452.
50. Zhao, J., Y. Lou, J. Pinczewski, N. Malkevitch, K. Aldrich, V. S. Kalyanaraman, D. Venzon, B. Peng, L. J. Patterson, Y. Edghill-Smith, R. Woodward, G. N. Pavlakis, and M. Robert-Guroff. 2003. Boosting of SIV-specific immune responses in rhesus macaques by repeated administration of Ad5hr-SIVenv/rev and Ad5hr-SIVgag recombinants. Vaccine 21:4022-4035.

## Claims

1. A method of preparing a recombinant adenovirus (RAdEs) vaccine to protect against Japanese encephalitis virus (JEV) infection, wherein said vaccine produces secretory envelop protein (Es) of JEV, said method comprising the steps of :
a. digesting plasmid pMEs from Japanese encephalitis virus with restriction enzymes *Kpn* I and *Bam* HI to obtain cDNA encoding JEV proteins prM and Es,
b. ligating the cDNA to adenovirus shuttle plasmid pShuttle digested with restriction enzymes *Kpn* I and *Hind* III at the *Kpn* I end,
c. adding nucleotides at the free *Bam* HI and *Hind* III ends with T4 DNA polymerase to create blunt ends,
d. ligating the blunt ends together to yield shuttle plasmid pSEs with JEV cDNA encoding the proteins prM and Es,
e. digesting the shuttle plasmid pSEs with restriction enzymes I-*Ceu* I and P1-*Sce* I to obtain expression cassette containing the JEV cDNA together with the CMV promoter/enhancer and BGH polyadenylation signal,
f. ligating the digested shuttle plasmid with I-*Ceu* I and P1-*Sce* I digested adenovirus plasmid pAdeno-X to generate plasmid pAdEs containing Es expression cassette,
g. digesting the plasmid pAdEs with *Pac* I,
h. transfecting the monolayers HEK 293 cells with digested plasmid pAdEs for about one week, and
i. obtaining the recombinant virus RAdEs vaccine.

2. A method as claimed in claim 1, wherein the transfection is at about 37°C temperature.

3. A method as claimed in claim 1, wherein the JEV proteins are under the control of human CMV IE promoter/enhancer.

4. A recombinant adenovirus (RAdEs) vaccine, comprising JEV Es protein prepared by the method of any one of claims 1 to 3 optionally with a pharmaceutically acceptable additive.

5. A vaccine as claimed in claim 4, wherein the vaccine produces secretory envelope protein of JEV.

6. A vaccine as claimed in claim 4 or 5, wherein the vaccine protects against Japanese encephalitis virus (JEV) infection.

7. A vaccine as claimed in any one claims 4 to 6, wherein the vaccine is effective by intra- muscular route of administration.

8. A vaccine as claimed in claim 4 or 5, wherein the additive is selected from a group comprising alum, gelatin and thiomersal.

9. A plasmid pAdEs of SEQ ID No. 1.

10. Use of a recombinant virus RAdEs vaccine comprising JEV Es protein according to claim 4 or prepared by the method of any one of claims 1 to 3 for preparing a medicament to protect a subject against Japanese encephalitis virus (JEV) infection.

11. Use as claimed in claim 10, wherein said medicament further comprises an additive.

12. Use as claimed in claim 10, wherein the medicament shows 100% efficacy.

13. Use as claimed in claim 10, for production of a medicament for protection against encephalitis.

14. Use as claimed in claim 10, wherein said medicament is formulated for administration to an animal.

15. Use as claimed in claim 10, wherein said medicament is formulated for administration to a human being.

16. Use as claimed in claim 10, wherein vaccine activates both humoral and cell-mediated immune response.

17. Use as claimed in claim 16, wherein the humoral response to the vaccine comprises IgG1 type of antibody.

18. Use as claimed in claim 10, wherein the vaccine leads to high amount of IFN-gamma secretion.

19. Use as claimed in claim 10, wherein the vaccine leads to moderate levels of IL-5 synthesis.

20. Use as claimed in claim 10, wherein an increased amount of the medicament leads to higher immune response.

21. Use as claimed in claim 10, wherein the medicament is more effective than commercially available vaccines.

## Patentansprüche

1. Verfahren zum Herstellen eines rekombinanten Vakzins von Adenovirus (RAdEs) zum Schutz gegen Infektion durch das Japanische Enzephalitis-Virus (JEV), bei welchem Verfahren das Vakzin sekretorisches Hüllprotein (Es) von JEV erzeugt, wobei das Verfahren die Schritte umfasst:
a. Digerieren von Plasmid pMEs aus Japanischem Enzephalitis-Virus mit den Restriktionsenzymen *Kpn* I und *Bam* HI, um cDNA zu erhalten, die die JEV-Proteine prM und Es codiert;
b. Ligieren der cDNA an das Adenovirus-Shuttle-Plasmid *pShuttle,* digeriert mit den Restriktionsenzymen *Kpn I* und *Hind III* an dem Ende von *Kpn* I;
c. Hinzufügen von Nucleotiden an den freien *Bam* HI- und *Hind* III-Enden mit T4 DNA-Polymerase, um Blunt-Enden zu erzeugen;
d. Ligieren der Blunt-Enden untereinander, um Shuttle-Plasmid pSEs mit JEV cDNA zu ergeben, die die Proteine prM und Es codiert;
e. Digerieren des Shuttle-Plasmids pSEs mit den Restriktionsenzymen I-*Ceu I* und PI-Sec I, um die Expressionskassette zu erhalten, welche die JEV cDNA enthält zusammen mit dem CMV Promotor/Enhancer und BGH Polyadenylierungssignal;
f. Ligieren des digerierten Shuttle-Plasmids mit I-*Ceu* I und PI-*Sec* I digeriertem Adenovirus-Plasmid pAdeno-X, um Plasmid pAdEs zu erzeugen, das Expressionskassette Es enthält;
g. Digerieren des Plasmid pAdEs mit *Pac* I;
h. Transfizieren der Monoschichten von HEK 293-Zellen mit digeriertem Plasmid pAdEs für etwa eine Woche, und
i. Gewinnen des rekombinanten Vakzins von Virus-RAdEs.

2. Verfahren nach Anspruch 1, wobei die Transfektion bei einer Temperatur von etwa 37°C erfolgt.

3. Verfahren nach Anspruch 1, wobei die JEV-Proteine unter der Kontrolle von humanem CMV IE Promotor/Enhancer stehen.

4. Rekombinantes Vakzin von Adenovirus (RAdEs), aufweisend JEV Es-Protein, das hergestellt ist mit Hilfe des Verfahrens nach einem der Ansprüche 1 bis 3 wahlweise mit einem pharmazeutisch unbedenklichen Additiv.

5. Vakzin nach Anspruch 4, wobei das Vakzin sekretorisches Hüllprotein von JEV erzeugt.

6. Vakzin nach Anspruch 4 oder 5, wobei das Vakzin gegen Infektion durch das Japanische Enzephalitis-Virus (JEV) schützt.

7. Vakzin nach einem der Ansprüche 4 bis 6, wobei das Vakzin durch Verabreichung auf dem intramuskulären Weg wirksam ist.

8. Vakzin nach Anspruch 4 oder 5, wobei das Additiv ausgewählt ist aus der Gruppe, bestehend aus: Alaun, Gelatine und Thiomersal.

9. Plasmid pAdEs von SEQ ID-No: 1.

10. Verwendung eines rekombinanten Vakzins von RadEs-Virus, aufweisend JEV Es-Protein nach Anspruch 4 oder hergestellt mit Hilfe des Verfahren nach einem der Ansprüche 1 bis 3 zum Herstellen eines Medikaments, um einen Probanden gegen Infektion durch das Japanische Enzephalitis-Virus (JEV) zu schützen.

11. Verwendung nach Anspruch 10, wobei das Medikament ferner ein Additiv aufweist.

12. Verwendung nach Anspruch 10, wobei das Medikament eine Wirksamkeit von 100 Prozent zeigt.

13. Verwendung nach Anspruch 10 zur Herstellung eines Medikaments für den Schutz gegen Enzephalitis.

14. Verwendung nach Anspruch 10, wobei das Medikament zur Verabreichung an ein Tier formuliert ist.

15. Verwendung nach Anspruch 10, wobei das Medikament zur Verabreichung an einen Menschen formuliert ist.

16. Verwendung nach Anspruch 10, wobei das Vakzin sowohl eine humorale als auch zellvermittelte Immunreaktion aktiviert.

17. Verwendung nach Anspruch 16, wobei die humorale Reaktion auf das Vakzin Antikörper vom Typ IgGI umfasst.

18. Verwendung nach Anspruch 10, wobei das Vakzin zu einer hohen Menge an Sekretion von IFN-gamma führt.

19. Verwendung nach Anspruch 10, wobei das Vakzin zu mäßigen Mengen an Synthese von IL-5 führt.

20. Verwendung nach Anspruch 10, wobei eine erhöhte Menge des Medikaments zu einer stärkeren Immunreaktion führt.

21. Verwendung nach Anspruch 10, wobei das Medikament wirksamer ist als kommerziell verfügbare Vakzine.

## Revendications

1. Procédé de préparation d'un vaccin à adénovirus recombiné (RAdEs) pour protéger contre l'infection par le virus de l'encéphalite japonaise (JEV), où ledit vaccin produit une protéine d'enveloppe sécrétoire (Es) de JEV, ledit procédé comprenant les étapes de :
a. digestion du plasmide pMEs du virus de l'encéphalite japonaise par les enzymes de restriction *Kpn* I et *Bam* HI pour obtenir l'ADNc codant les protéines prM et Es de JEV,
b. ligature de l'ADNc au plasmide navette de l'adénovirus pshuttle digéré par les enzymes de restriction *Kpn* I et *Hind* III à l'extrémité *Kpn* I,
c. addition de nucléotides aux extrémités libres *Bam* HI et *Hind* III au moyen de T4 ADN polymérase pour créer des extrémités franches,
d. ligature des extrémités franches entre elles pour obtenir le plasmide navette pSEs avec l'ADNc de JEV codant les protéines prM et Es,
e. digestion du plasmide navette pSEs par les enzymes de restriction I-*Ceu* I et PI-*Sce* I pour obtenir une cassette d'expression contenant l'ADNc de JEV avec le promoteur/activateur de CMV et le signal de polyadénylation de BGH,
f. ligature du plasmide navette digéré avec le plasmide d'adénovirus pAdeno-X digéré par I-*Ceu* I et PI-*Sce* I pour produire le plasmide pAdEs contenant la cassette d'expression de Es,
g. digestion du plasmide pAdEs par *Pac*I,
h. transfection des monocouches de cellules HEK 293 par le plasmide pAdEs digéré pendant environ 1 semaine et
i. obtention du vaccin à virus recombiné RAdEs.

2. Procédé selon la revendication 1, où la transfection est effectuée à une température d'environ 37°C.

3. Procédé selon la revendication 1, où les protéines de JEV sont sous le contrôle du promoteur/activateur IE de CMV humain.

4. Vaccin à adénovirus recombiné (RAdEs), comprenant la protéine Es de JEV préparée par le procédé selon l'une quelconque des revendications 1 à 3 facultativement avec un additif pharmaceutiquement acceptable.

5. Vaccin selon la revendication 4, où le vaccin produit une protéine d'enveloppe sécrétoire de JEV.

6. Vaccin selon la revendication 4 ou 5, où le vaccin protège contre l'infection par le virus de l'encéphalite japonaise (JEV).

7. Vaccin selon l'une quelconque des revendications 4 à 6, où le vaccin est efficace par la voie d'administration intramusculaire.

8. Vaccin selon la revendication 4 ou 5, où l'additif est sélectionné dans un groupe comprenant l'alun, la gélatine et le thiomersal.

9. Plasmide pAdEs de SEQ ID NO:1.

10. Utilisation d'un vaccin à virus recombiné RAdEs comprenant la protéine Es de JEV selon la revendication 4 ou préparé par le procédé selon l'une quelconque des revendications 1 à 3 pour préparer un médicament destiné à protéger un sujet contre l'infection par le virus de l'encéphalite japonaise (JEV).

11. Utilisation selon la revendication 10, où ledit médicament comprend en outre un additif.

12. Utilisation selon la revendication 10, où le médicament présente une efficacité de 100 %.

13. Utilisation selon la revendication 10 pour la production d'un médicament destiné à protéger contre l'encéphalite.

14. Utilisation selon la revendication 10, où ledit médicament est formulé en vue de l'administration à un animal.

15. Utilisation selon la revendication 10, où ledit médicament est formulé en vue de l'administration à un être humain.

16. Utilisation selon la revendication 10, où le vaccin active la réponse immunitaire à médiation humorale et à médiation cellulaire.

17. Utilisation selon la revendication 16, où la réponse humorale au vaccin comprend le type d'anticorps IgGI.

18. Utilisation selon la revendication 10, où le vaccin entraîne une grande quantité de sécrétion d'IFN-gamma.

19. Utilisation selon la revendication 10, où le vaccin entraîne des taux modérés de synthèse de IL-5.

20. Utilisation selon la revendication 10, où une quantité accrue du médicament entraîne une réponse immunitaire supérieure.

21. Utilisation selon la revendication 10, où le médicament est plus efficace que les vaccins du commerce.
